(19) Europäisches Patentamt
European Patent Office
Office européen des brevets

(11) Numéro de publication: **0 192 002 B1**

(12) **FASCICULE DE BREVET EUROPEEN**

(45) Date de publication de fascicule du brevet: **29.04.92**

(51) Int. Cl.[5]: **C07K 15/14**, A61K 39/395, //A61K47/00

(21) Numéro de dépôt: **85402403.1**

(22) Date de dépôt: **04.12.85**

(54) **Immunotoxines à longue durée d'action comportant un constituant glycopeptique inactivant les ribosomes modifié sur ses motifs polysaccharidiques.**

(30) Priorité: **13.02.85 FR 8502068**

(43) Date de publication de la demande:
**27.08.86 Bulletin 86/35**

(45) Mention de la délivrance du brevet:
**29.04.92 Bulletin 92/18**

(84) Etats contractants désignés:
**AT BE CH DE FR GB IT LI LU NL SE**

(56) Documents cités:
**EP-A- 0 023 401** **EP-A- 0 044 167**
**EP-A- 0 074 279** **EP-A- 0 080 401**
**EP-A- 0 088 695** **EP-A- 0 172 045**
**FR-A- 2 312 259** **FR-A- 2 437 213**

**EUROPEAN JOURNAL OF BIOCHEMISTRY, vol. 147, no. 1, 1985, pages 197-206; P.E. THORPE et al.: "Modification of the carbohydrate in ricin with metaperiodate - cyanoborohydride mixtures"**

(73) Titulaire: **SANOFI S.A.**
**40, Avenue George V**
**F-75008 Paris(FR)**

(72) Inventeur: **Jansen, Franz**
**Chemin des Fresquets Assas**
**F-34160 Castries(FR)**
Inventeur: **Gros, Pierre**
**18, rue des Muriers**
**F-34100 Montpellier(FR)**

(74) Mandataire: **Gillard, Marie-Louise et al**
**Cabinet Beau de Loménie 55, Rue d'Amsterdam**
**F-75008 Paris(FR)**

**EP 0 192 002 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouvelles molécules médicamenteuses comportant au moins un anticorps lié de façon covalente à un constituant de nature polypeptidique, inhibiteur de la synthèse protéique et issu d'une glycoprotéine (ou d'un glycopeptide) dont les motifs polysaccharidiques ont été modifiés.

Dans le brevet US 4 340 535 et dans les demandes de brevet français n° 81/07596 et n° 81/21836 on a décrit la préparation de produits anti-cancéreux dits conjugués obtenus par couplage, par liaison covalente, de la chaîne A de ricine avec des anticorps ou fragments d'anticorps dirigés contre des antigènes portés par la cellule à détruire. Les produits de ce type ont été désignés et sont désignés dans la présente demande sous le nom générique d'Immunotoxines. Des conjugués du même type ont été également décrits dans le brevet EP-A- 023.401.

On connaît aussi des conjugués analogues aux immunotoxines à chaîne A de ricine précédemment décrites, ayant aussi vocation de médicaments anti-cancéreux et résultant du couplage par liaison covalente d'anticorps ou fragments d'anticorps à d'autres glycoprotéines inactivant les ribosomes tels que notamment la gélonine extraite de Gelonium multiflorum (Eur. J. Biochem. 1981, 116, 447-454, Cancer Res. 1984, 44, 129-133) ou l'inhibiteur extrait de Momordica charantia (MOM) (brevet US 4 368 149).

Ces glycoprotéines inactivant les ribosomes (abrégé GPIR) qui ont des propriétés semblables à celles de la chaîne A de ricine sont des substances de poids moléculaire d'un ordre de grandeur de 20 000 et 30 000 (Cancer Survey, 1982, 1, 489-520).

Le terme "glycoprotéine inactivant les ribosomes", tel qu'utilisé dans la présente description ainsi que dans les revendications, désigne toute substance portant des motifs saccharidiques appartenant à la classe des macromolécules inactivant les ribosomes et, par conséquent, inhibant la synthèse protéique de cellules eucaryotes, ainsi que tout fragment de ladite substance possédant la même propriété inactivante, ladite glycoprotéine inactivant les ribosomes pouvant être d'origine naturelle ou biosynthétique provenant d'une cellule dont le patrimoine génétique a été modifié dans ce but.

On sait également que l'activité cytotoxique de ces immunotoxines peut être potentialisée par diverses substances adjuvantes telles que les sels d'ammonium, diverses amines ou certains ionophores carboxyliques tels que la monensine ou la nigéricine.

Toutefois, les effets thérapeutiques des immunotoxines, activées ou non, ne peuvent se manifester pleinement que dans la mesure où l'immunotoxine peut, par sa partie anticorps, se localiser in vivo, sous forme active, sur la cible cellulaire à détruire (condition sine qua non à toute expression d'activité des immunotoxines). La capacité de l'immunotoxine à se localiser sur la cible dépend en tout premier lieu de l'aptitude de l'immunotoxine à demeurer dans la circulation sanguine et les fluides extra-cellulaires sous forme active pendant des temps suffisants pour qu'elle atteigne sa cible cellulaire et à des concentrations suffisamment fortes pour que le taux d'occupation des sites antigéniques correspondants soit élevé.

De nombreuses études ont permis d'établir la cinétique d'élimination plasmatique des immunotoxines après injection intraveineuse dans différents modèles animaux. Il est apparu qu'après l'injection le taux plasmatique d'immunotoxine biologiquement active décroît très rapidement et de façon très importante. Ainsi, typiquement, chez le lapin, dans un modèle utilisant une immunotoxine construite en couplant, à l'aide d'un bras à pont disulfure, la chaîne A de ricine à un anticorps monoclonal dirigé contre l'antigène T65 des lymphocytes T humains (anticorps T101), il apparaît que 97 % de l'immunotoxine présente dans le sang circulant au temps 0 de l'injection disparaissent en 30 min et 99,9 % en 17 h. Cette rapide disparition de l'immunotoxine est bien évidemment préjudiciable à l'expression de sa capacité cytotoxique complète, en empêchant que l'immunotoxine ne sature de façon durable une proportion élevée des antigènes-cibles portés par les cellules à détruire. En outre, la comparaison des cinétiques d'élimination plasmatique des immunotoxines avec celles des anticorps non conjugués correspondants montre qu'à l'inverse les anticorps - comme cela est bien connu - se maintiennent dans le plasma à un haut niveau pendant des temps relativement longs. Or il existe toujours, même dans les préparations d'immunotoxines les plus purifiées, un certain taux résiduel d'anticorps non conjugués. Par le jeu des vitesses différentielles d'élimination des immunotoxines et des anticorps, progressivement les anticorps non conjugués, initialement très minoritaires, deviennent majoritaires au bout de quelques heures et donc, progressivement, ces anticorps deviennent par compétition de puissants antagonistes pour la fixation des immunotoxines sur leurs cibles.

Il apparaît donc clairement l'intérêt d'accroître la persistance plasmatique des immunotoxines, sous forme active, afin d'augmenter à la fois la durée et le taux d'occupation des antigènescibles et par conséquent d'améliorer les effets thérapeutiques des immunotoxines.

Par ailleurs, des expériences de localisation in vivo de l'immunotoxine à chaîne A de ricine, radiomarquée puis injectée chez des animaux sans cible spécifique,ont montré que, dans les premières minutes après l'injection, le conjugué se localise préférentiellement dans le foie. Il en est de même pour la chaîne A de ricine qui présente le même devenir lorsqu'elle est injectée sous forme non couplée. Cela suggère fortement que l'immunotoxine se fixe dans le foie par l'intermédiaire de la sous-unité cytotoxique qu'elle contient.

Il est connu que la chaîne A de ricine est une glycoprotéine dont les groupements polyosidiques comprennent notamment des résidus de mannose et de N-acétylglucosamine, certains résidus de mannose étant en positions terminales (Agri. Biol. Chem., 1978, 42, 501). Il a été également établi l'existence au niveau du foie de récepteurs capables de reconnaître des glycoprotéines ayant de tels résidus de mannose terminaux. Il a été aussi montré que les glycoprotéines reconnues par ces récepteurs - présents essentiellement sur les cellules de Kupffer - sont rapidement éliminées de la circulation sanguine par fixation sur ces cellules qui les métabolisent. Ceci est bien documenté notamment dans le cas de la bêtaglucuronidase, ainsi que dans le cas de la ribonucléase B (Arch. Biochem. Biophys., 1978, 188, 418 ; Advances in Enzymology, Meister A. Ed., New York, 1974 ; Pediat. Res., 1977, 11, 816).

De l'ensemble de ces données, il apparaît que la rapide élimination des immunotoxines à chaîne A de ricine peut s'expliquer par la reconnaissance des résidus mannose de la chaîne A de ricine par les cellules hépatiques et en particulier les cellules de Kupffer.

Les études de cinétique d'élimination plasmatique d'autres GPIR, par exemple la gélonine ou le MOM, après injection intraveineuse chez l'animal, ont montré que, comme dans le cas de la chaîne A de ricine, le taux plasmatique de GPIR décroît très rapidement et de façon très importante après l'injection. Ainsi typiquement chez le lapin après injection de gélonine purifiée selon la méthode décrite (J. Biol. Chem., 1980, 255, 6947-6953) il apparaît que 93 % de la gélonine présente dans le sang circulant au temps 0 de l'injection disparaissent en 1 h et 99,99 % en 24 h.

Il est connu que par oxydation par les ions periodate des structures osidiques, y compris celles qui sont contenues dans les glycoprotéines, on provoque la rupture de la chaîne carbonée chaque fois que deux atomes de carbone adjacents portent des hydroxyles primaires ou secondaires. Si les deux hydroxyles contigus sont secondaires, comme cela est généralement le cas dans les oses cycliques présents dans les GPIR, l'oxydation conduit à deux fonctions aldéhyde sur les carbones entre lesquels la rupture a eu lieu.

Dans la description le terme "periodate" désigne l'ion IO-4 que l'on trouve également indiqué dans la littérature sous le nom de "métaperiodate".

On a maintenant trouvé, d'une façon absolument inattendue, que, si on modifie les motifs glucidiques d'une glycoprotéine inactivant les ribosomes par une oxydation par les ions periodate, on obtient une nouvelle glycoprotéine inactivant les ribosomes ayant la double propriété de conserver ses activités biologiques et d'être éliminée très lentement de la circulation sanguine in vivo.

On a également trouvé que lorsque ces nouvelles glycoprotéines inactivant les ribosomes et à longue durée d'action sont couplées à des anticorps, les conjugués obtenus conservent les propriétés biologiques connues des immunotoxines et présentent une cinétique d'élimination plasmatique lente.

On notera que dans l'art antérieur, l'oxydation periodique a été proposée pour la fabrication de conjugués constitués d'un anticorps ou d'un fragment d'anticorps et d'une substance antitumorale. Dans ce cas, les groupes aldéhydiques obtenus par oxydation periodique de la substance antitumorale sont utilisés pour réaliser le conjugué souhaité. Ces groupes aldéhydiques sont donc introduits temporairement pour permettre la conjugaison anticorps-substance antitumorale. A cet effet, on peut citer les brevets EP-A-0 074 279, EP-A-0 088 695, EP-A-0 044 167 et le brevet FR-A 2 312 259 dans lesquels les glycoprotéines mises en oeuvre dans la présente demande ne sont pas citées.

Au contraire, dans la présente demande, les fonctions aldéhyde résultant de l'oxydation periodique de la glrcoprotéine ne sont pas impliquées dans la réaction de couplage entre l'anticorps ou le fragment d'anticorps et ladite glycoprotéine, pour former les immunotoxines selon l'invention.

La présente invention a donc pour objet des produits appartenant à la classe des immunotoxines, obtenus par couplage, par une structure covalente divalente contenant un groupe disulfure ou thioéther, entre d'une part un anticorps ou fragment d'anticorps, utilisé sous forme naturelle ou correctement modifié, et d'autre part une glycoprotéine inactivant les ribosomes, dont les motifs glucidiques sont modifiés par oxydation par l'ion periodate, les fonctions aldéhyde résultant de cette oxydation n'étant pas impliquées dans le couplage ci-dessus.

Pour des raisons de clarté, on précise ci-après la signification des symboles utilisés pour désigner les protéines différentes ou leurs radicaux et des expressions utilisées pour désigner les différents symboles.

Le symbole P représente une protéine choisie parmi tout anticorps ou fragment d'anticorps, ou toute immunoglobuline ou fragment d'immunoglobuline ou toute molécule dérivant des précédentes par modification artificielle de l'un quelconque de leurs groupements fonctionnels, y compris des structures glucidiques qu'elles portent, sous réserve que la protéine ainsi choisie reste capable de reconnaître sélectivement un antigène donné à la surface des cellules porteuses de cet antigène et notamment des cellules cibles. La protéine P de départ peut être d'origine naturelle ou biosynthétique provenant d'une cellule dont le patrimoine génétique a été modifié dans ce but.

Le symbole GPIR-la représente une protéine qui est une glycoprotéine inactivant les ribosomes et à longue durée d'action, obtenue par traitement d'une glycoprotéine inactivant les ribosomes, dont les groupes thiol sont éventuellement protégés, avec une solution aqueuse d'un periodate alcalin pendant une période de temps de 0,2 à 24 h à la température de 0 à 15ºC et à l'abri de la lumière et par déblocage éventuel des groupes thiol, ou toute molécule dérivant de celle-ci par modification artificielle de tout groupement fonctionnel porté par cette protéine, sous réserve que la protéine ainsi choisie présente encore la propriété d'inhiber la synthèse protéique ribosomale des cellules eucaryotes telle que l'on peut la mettre en évidence dans un modèle d'étude acellulaire. Dans l'immunotoxine, la partie GPIR-la est également indiquée comme "sous-unité cytotoxique".

Le symbole A-la représente une glycoprotéine inactivant les ribosomes et à longue durée d'action, obtenue par traitement de la chaîne A de ricine, dont au moins un des groupes thiol de ses cystéines 171 et 257 est éventuellement protégé, avec une solution aqueuse d'un periodate alcalin pendant une période de temps de 0,2 à 24 h à la température de 0 à 15 ° C et à l'abri de la lumière et par déprotection éventuelle dudit groupe thiol.

Le symbole P' représente un radical dérivé de la protéine P ci-dessus telle quelle ou chimiquement convenablement modifiée, privée d'une ou plusieurs fonctions propres et dont d'autres groupes fonctionnels sont éventuellement bloqués.

Le symbole GPIR-la' représente un radical dérivé de la protéine GPIR-la ci-dessus telle quelle ou chimiquement convenablement modifiée, privée d'une ou plusieurs fonctions propres et dont d'autres groupes fonctionnels sont éventuellement bloqués.

Le symbole A-la' représente un radical dérivé de la protéine A-la privé d'au moins une des fonctions thiol de ses cystéines 171 et 257.

Le symbole $P_1$ représente une des protéines GPIR-la et P telles que définies ci-dessus qui porte des fonctions thiol libres, attachées à ladite protéine directement ou par l'intermédiaire d'une structure d'espacement.

Le symbole $P_2$, différent de $P_1$, représente une des protéines GPIR-la et P telles que définies ci-dessus, qui porte un ou plusieurs groupements fonctionnels capables de réagir avec les thiols libres.

Le symbole $P_1$, représente le radical de la protéine $P_1$ lié aux fonctions propres de la protéine $P_1$ notamment les fonctions SH (de la cystéine), $NH_2$ (terminal de la protéine ou dans la position epsilon des lysines), OH (des tyrosines), COOH (des acides aspartiques et glutamiques) ou, seulement dans le cas où $P_1$ est un anticorps ou fragment d'anticorps, le radical de la protéine $P_1$ provenant de l'ouverture des structures glucidiques par action de l'acide periodique selon des méthodes connues.

Le symbole $P_2$, représente le radical de la protéine $P_2$ lié aux groupes fonctionnels caractéristiques $NH_2$ (terminal de la protéine ou dans la position epsilon des lysines), OH (des tyrosines), COOH (des acides aspartiques et glutamiques).

Par exemple, $P_1$,-SH représente la protéine $P_1$ (qui peut être indifféremment l'anticorps ou fragment d'anticorps P ou la GPIR-la) dans laquelle les groupes SH des cystéines sont libres et les autres groupes fonctionnels sont éventuellement bloqués.

De la même façon, $P_1$,-CO- représente la protéine $P_1$, dans laquelle son groupe carboxylique terminal ou les groupes carboxyliques de ses acides glutamiques et aspartiques sont couplés avec un groupement qui apporte un groupe SH introduit artificiellement.

Encore $P_2$,-NH- représente la protéine $P_2$ (qui peut être indifféremment l'anticorps ou fragment d'anticorps P ou la GPIR-la) dans laquelle son groupe amino terminal ou les groupes amino de ses lysines sont attachés à un groupement susceptible de couplage avec le thiol de la protéine $P_1$.

Le terme "structure d'espacement inerte" tel qu'utilisé ici pour E et E' désigne un radical organique bivalent inerte vis-à-vis des réactifs utilisés dans le procédé, tel qu'un groupe alkylène à chaîne droite ou ramifiée contenant de 1 à 15 atomes de carbone, qui peut contenir une ou plusieurs doubles liaisons ou qui peut être interrompu par des atomes d'oxygène ou qui peut être substitué par un ou plusieurs groupes fonctionnels inertes, tels que des groupes méthoxy, des groupes carboxyles libres ou estérifiés, des

groupes dialkylamino, des groupes carbamates. Le même terme désigne également un groupe arylène contenant de 6 à 15 atomes de carbone qui peut être substitué par un ou plusieurs groupes fonctionnels inertes comme indiqué ci-dessus pour le groupe alkylène.

L'expression "groupe fonctionnel capable de se lier d'une façon covalente" telle qu'utilisée ici pour Y et Y' désigne tous les groupes susceptibles de réagir avec les fonctions propres des protéines $P_1$ et $P_2$ pour donner une liaison covalente. Ainsi, les groupes -CO- et -(C=NH)- sont des groupes fonctionnels, convenables susceptibles de se lier aux amines libres, les thiols et les hydroxyles phénoliques des protéines. De même, le groupe -NH- est un groupe fonctionnel convenable, susceptible de se lier aux groupes carboxyliques libres des protéines. Le groupe =N- est un groupe fonctionnel convenable, susceptible de se lier aux deux atomes de carbone des structures glucidiques des protéines $P_1$ et $P_2$ après oxydation avec les ions periodate, mais seulement dans le cas où $P_1$ et $P_2$ sont un anticorps ou un fragment d'anticorps.

L'expression "fonction propre des protéines", telle qu'utilisée ici pour Z et Z', désigne les radicaux provenant des groupes caractéristiques des acides aminés formant les protéines $P_1$ et $P_2$, telles que l'atome d'oxygène provenant des hydroxyles des acides aminés tyrosine et éventuellement sérine, le groupe carbonyle provenant du carboxyle terminal ou des carboxyles libres des acides aspartique et glutamique, le groupe -NH- provenant de l'amine terminale des protéines par exemple de la lysine, l'atome de soufre provenant du thiol de la cystéine. La même expression désigne également le groupe provenant de la structure dialdéhydique obtenue après oxydation d'une des structures glucidiques des protéines $P_1$ et $P_2$ par traitement avec les ions periodate, mais seulement dans le cas où les $P_1$ et $P_2$ sont un anticorps ou fragment d'anticorps.

Le terme "radical activateur", tel qu'utilisé ici pour X, désigne un groupement lié à un pont -S-S- capable de réagir avec un thiol libre pour former un disulfure avec libération de X-SH. Des radicaux activateurs convenables sont les groupes pyridyl-2 et pyridyl-4, non substitués ou substitués par un ou des halogènes ou des radicaux alkyle, carboxyle, alcoxycarbonyle ; le groupe phényle non substitué ou, de préférence, substitué par un ou des halogènes ou des groupes nitro, alcoxy, carboxyle ou alcoxycarbonyle ; ou un groupe alcoxycarbonyle tel que méthoxycarbonyle.

Les termes "alkyle" et "alcoxy" désignent des groupes contenant jusqu'à 5 atomes de carbone.

Le terme "alkylène" désigne des groupements aliphatiques saturés, à chaîne droite ou ramifiée, contenant jusqu'à 10 atomes de carbone pouvant être substitués par un ou plusieurs groupes fonctionnels inertes tels que les groupes alcoxycarbonyle.

Plus particulièrement, la présente invention a pour objet des produits, appartenant à la classe des Immunotoxines, obtenus par couplage covalent entre, d'une part un anticorps ou fragment d'anticorps, utilisé sous sa forme naturelle ou correctement modifié (symbole P), possédant la capacité de reconnaître sélectivement un antigène porté par les cellules-cibles visées et, d'autre part, une glycoprotéine inactivant les ribosomes et à longue durée d'action, obtenue par traitement d'une glycoprotéine inactivant les ribosomes, dont les groupes thiol sont éventuellement protégés avec une solution aqueuse d'un periodate alcalin pendant une période de temps de 0,2 à 24 h à la température de 0 à 15 °C et à l'abri de la lumière et par déblocage éventuel des groupes thiol (symbole GPIR-la), le couplage entre les 2 protéines étant réalisé soit par l'intermédiaire d'une liaison disulfure soit par l'intermédiaire d'une liaison thioéther.

Une immunotoxine formée par couplage d'un anticorps P avec une glycoprotéine inactivant les ribosomes et à longue durée d'action GPIR-la peut être représentée par la formule statistique suivante :

P'-W-GPIR-la' I

dans laquelle P' représente le radical d'une protéine qui est un anticorps ou un fragment d'anticorps P tel quel ou chimiquement convenablement modifié et dans lequel d'autres groupes fonctionnels sont éventuellement bloqués, GPIR-la' représente le radical d'une protéine qui est la GPIR-la telle quelle ou chimiquement convenablement modifiée et dans lequel d'autres groupes fonctionnels sont éventuellement bloqués et W représente une structure covalente bivalente contenant un groupe thioéther ou un groupe disulfure dont les atomes de soufre soit sont ceux des cystéines de P et GPIR-la soit sont liés aux fonctions propres de P et/ou de GPIR-la par des structures d'espacement portant un groupe fonctionnel lié auxdites fonctions propres de P et/ou de GPIR-la.

Par liaison thioéther entre deux protéines on entend une liaison du type :

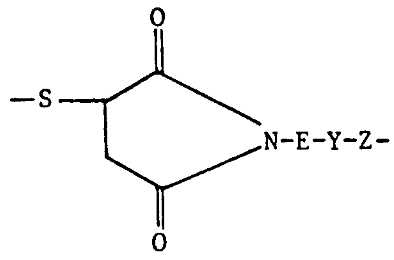

dans laquelle Z, Y et E sont tels que définis ci-dessous.

D'une façon préférentielle, la présente invention concerne une immunotoxine de formule statistique :

P'-W'-GPIR-la' II

dans laquelle P' et GPRI-la' sont tels que définis ci-dessus et W' représente une structure covalente choisie parmi :

(a) un groupement de formule

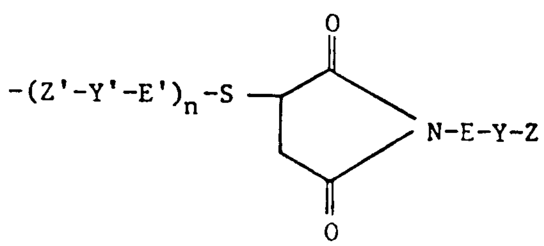

(b) un groupement de formule

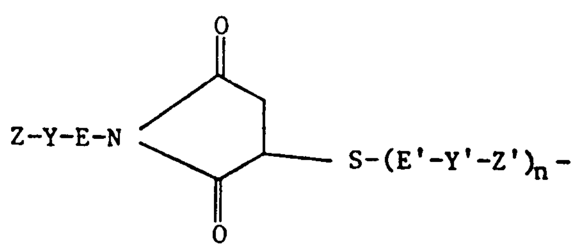

(c) un groupement de formule

$-Z-Y-E-S-S-(E'-Y'-Z')_n-$

(d) un groupement de formule

$-(Z'-Y'-E')_n-S-S-E-Y-Z-$

où

- Z et Z' représentent les fonctions propres des protéines GPIR-la et P, choisies parmi l'atome d'oxygène provenant de l'hydroxyle de l'un des résidus de tyrosine ; le groupe carbonyle provenant de l'un des carboxyles terminaux ou des carboxyles libres des acides aspartiques et/ou glutamiques de GPIR-la et de P ; le groupe -NH- provenant de l'une des amines terminales de GPIR-la et de P ou de l'une des amines en position epsilon de l'un des résidus de lysine ; et seulement pour Z dans les structures covalentes (b) et (c), le groupe provenant de la structure dialdéhydique obtenue après oxydation d'une des structures glucidiques de P par l'acide periodique selon les méthodes connues.

- Y et Y' représentent des groupes fonctionnels capables de se lier d'une façon covalente avec l'une quelconque des fonctions Z et Z' des protéines GPIR-la et P ;
- E et E' représentent des structures d'espacement inertes ; et
- n représente zéro ou 1.

Les immunotoxines telles que représentées par les formules I et II ci-dessus le sont, en forme simplifiée, mais il est entendu que la structure covalente bivalente -W- ou -W'- est liée à au moins une molécule P et au moins une molécule GPIR-la. Le nombre de liaisons avec les protéines P et GPIR-la dépend du nombre des fonctions propres desdites protéines intervenant dans l'opération de couplage.

Par exemple, si une immunotoxine est formée par couplage de la sous-unité A de la ricine native avec l'anticorps P (par exemple l'anticorps T101) par l'intermédiaire d'une structure covalente bivalente ayant un groupe disulfure dont un soufre est celui de la cystéine 257 de la chaîne A de la ricine à longue durée d'action et l'autre est lié aux oxygènes phénoliques des tyrosines de l'anticorps P par un groupe oxopropylique, elle aura la formule statistique

$P'(O\text{-}CO\text{-}CH_2\text{-}CH_2\text{-}S\text{-}S\text{-}A\text{-}la')t$

dans laquelle t représente le nombre des tyrosines de l'anticorps (par exemple de l'anticorps T101) intervenues dans le couplage.

L'immunotoxine ainsi obtenue correspond à un produit de formule II, où :

- P' est tel que défini ci-dessus, notamment le radical de l'anticorps T101 privé des fonctions phénoliques des tyrosines, intervenant dans le couplage ;
- A-la' le radical de la chaîne A de la ricine à longue durée d'action, privée de la fonction thiol de sa cystéine 257 ;
- W' est le groupement (c) :

$-Z\text{-}Y\text{-}E\text{-}S\text{-}S\text{-}(E'\text{-}Y'\text{-}Z')_n\text{-}$

où Z est l'oxygène des hydroxyles phénoliques intervenus dans le couplage, Y est -CO- ; E est la structure d'espacement inerte $-CH_2\text{-}CH_2\text{-}$ et n est zéro.

Particulièrement préférées sont les immunotoxines formées par une ou plusieurs structures contenant la sous-unité A de la ricine à longue durée d'action et un seul anticorps P, représentées par la formule statistique

P'(W'-A-la')m III

dans laquelle P', W' et A-la' sont tels que définis ci-dessus et m représente le nombre de fonctions propres de la protéine P intervenues dans le couplage. Le nombre m varie de 0,3 à 12, de préférence de 0,5 à 10.

L'expression "m varie de 0,3 à 12, de préférence de 0,5 à 10" signifie que la valeur de m est statistique car dans la population des molécules d'anticorps le couplage ne se produit pas d'une façon homogène. Le nombre m peut donc ne pas être entier.

La valeur de m dépend notamment des anticorps utilisés, plus particulièrement de leur poids moléculaire.

Ainsi, si comme anticorps P de départ on utilise un fragment Fab ou Fab', la valeur de m pourra varier entre 0,3 et environ 2 ; si l'on utilise un fragment F(ab')2, m pourra varier entre 0,5 et environ 4 ; pour un anticorps du type IgG, la valeur de m sera entre 0,5 et environ 6 ; enfin, pour un anticorps IgM, la valeur de m pourra varier entre 1 et environ 12.

Il est cependant préférable que le degré de substitution sur l'anticorps P soit tel qu'il conduise à une valeur de m non inférieure à 0,5 et non supérieure à 10.

Plus généralement, les structures I et II ci-dessus représentent des formules statistiques écrites de façon simplifiée, comme il a été spécifié ci-dessus.

D'une façon analogue, les formules IV, V et IX ci-dessous sont également statistiques - lorsque, le cas échéant, n est 1 - car les réactifs de couplage sont préparés à partir de populations de protéines $P_1$ et $P_2$ qui ont toutes exactement les mêmes propriétés que celles prises en compte ci-dessus pour l'anticorps P, que ces protéines $P_1$ et $P_2$ soient elles-mêmes l'anticorps P ou la GPIR-la.

Selon un autre de ses aspects, la présente invention concerne un procédé pour la préparation d'une immunotoxine à longue durée d'action ayant une liaison covalente du type disulfure ou thioéther entre un anticorps et une glycoprotéine inactivant les ribosomes, caractérisé en ce que l'on forme une liaison disulfure ou thioéther entre un anticorps et une glycoprotéine inactivant les ribosomes à longue durée

d'action obtenue par traitement d'une glycoprotéine inactivant les ribosomes, dont les groupes thiol sont éventuellement protégés avec une solution aqueuse d'une periodate alcalin pendant une période de temps de 0,2 à 24 h à la température de 0 à 15° C et à l'abri de la lumière et par déblocage éventuel du groupe thiol.

Selon un aspect préférentiel, la présente invention concerne un procédé pour la préparation d'une immunotoxine ayant la structure I ci-dessus, caractérisé en ce qu'on fait réagir en solution aqueuse et à la température ambiante une protéine $P_1$ qui est indifféremment soit la glycoprotéine inactivant les ribosomes à longue durée d'action GPIR-la, soit un anticorps ou fragment d'anticorps, porteuse d'au moins un groupe thiol libre attaché à ladite protéine $P_1$ directement ou par l'intermédiaire d'une structure d'espacement, avec une protéine $P_2$, différente de $P_1$, qui est indifféremment soit la glycoprotéine inactivant les ribosomes à longue durée d'action GPIR-la, soit un anticorps ou fragment d'anticorps, porteuse d'un groupement capable de couplage avec le thiol libre de la protéine $P_1$, de façon à former une liaison thioéther ou disulfure.

Selon un aspect particulièrement avantageux, la présente invention concerne un procédé pour la préparation d'une immunotoxine ayant la structure II, dans laquelle P', W' et GPIR-la' sont tels que définis ci-dessus, caractérisé en ce qu'on fait réagir en solution aqueuse et à la température ambiante une protéine de formule

$P_{1'}$-(Z-Y-E)$_n$-SH IV

avec une protéine de formule statistique

$P_{2'}$-Z'-Y'-E'-G V

où $P_{1'}$ et $P_{2'}$ représentent les radicaux des protéines $P_1$ et $P_2$ liés aux fonctions propres desdites protéines ou, seulement lorsque $P_1$ et $P_2$ sont un anticorps ou fraction d'anticorps, les radicaux des protéines $P_1$ et $P_2$ provenant de l'ouverture des structures glucidiques par action de l'acide periodique, Z, Z', Y, Y', E et E' sont tels que définis ci-dessus et G représente un groupe

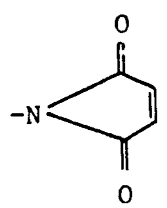

ou un groupe -S-S-X, où X est un groupe activateur.

Tant P que GPIR-la sont donc des protéines qui présentent indifféremment

(1) la ou les fonctions thiol qui participent au couplage et

(2) un ou plusieurs groupements fonctionnels capables de réagir avec les fonctions thiol ci-dessus pour former une liaison disulfure ou thioéther.

Selon la présente invention lesdites fonctions thiol et groupements fonctionnels sont ceux des protéines P ou GPIR-la natives, ou bien ils y sont introduits artificiellement.

Les glycoprotéines inactivant les ribosomes utilisés comme produit de départ pour l'oxydation periodique selon l'invention sont toutes les GPIR comme la chaîne A de ricine, qui sont par elles-mêmes très faiblement cytotoxiques car elles ne peuvent se fixer sur les cellules, mais qui, par contre, couplées à un anticorps, reconnaissant des cellules particulières, deviennent hautement cytotoxiques pour ces cellules une fois que l'anticorps a reconnu sa cible.

Des composés de départ représentatifs sont la chaîne A de ricine, la gélonine et la substance extraite de Momordica charantia (MOM) telles qu'elles sont obtenues par la voie extractive.

D'autres GPIR utiles comme produit de départ pour l'oxydation par les ions periodates sont les suivantes :

Liste

| | |
|---|---|
| Dianthin 30 | de Dianthus caryophyllus |
| Dianthin 32 | de " " |
| Agrostin A | de Agrostemma githago |
| Agrostin B | de " " |
| Agrostin C | de " " |

| | |
|---|---|
| HCI | de Hura crepitans |
| Asparagus officinalis inhibitor | de Asparagus officinalis |

Les mêmes substances, produites par voie biosynthétique par des cellules dont le patrimoine génétique a été modifié dans ce but sont des composés également convenables.

Des fragments des GPIR ci-dessus, à condition que de tels fragments retiennent tout ou partie de la propriété d'inactivation des ribosomes qui caractérise la GPIR dont ils sont issus, peuvent également être utilisés comme produits de départ.

La chaîne A de ricine native, dont au moins l'un des groupements thiol est protégé, est un composé de départ préférentiel.

L'obtention de la chaîne A de ricine pure est décrite dans le brevet US 4 340 535. La gélonine et le MOM sont également décrits.

La protection des groupes thiol des GPIR de départ n'est nécessaire que lorsque lesdits groupes thiol sont ceux qui seront utilisés pour le couplage avec l'anticorps. Si pour le couplage on utilise d'autres groupes fonctionnels, par exemple l'hydroxyle phénolique des tyrosines, la protection n'est pas effectuée.

Le blocage est effectué par réaction avec un agent susceptible de substituer les fonctions SH à l'aide d'un radical qui peut être ensuite éliminé par réduction ou échange thiol/disulfure, par exemple l'acide dinitro-2,2' dithio-5,5' dibenzoïque (DTNB) ou bien par l'acide (pyridyl-2 disulfanyl)-3 propionique. En l'absence d'un tel traitement les thiols libres de la chaîne A peuvent disparaître pendant la réaction d'oxydation et dans ce cas ne peuvent être totalement régénérés par l'action d'un agent réducteur tel que le mercapto-2 éthanol. L'excès de l'agent bloquant est éliminé par dialyse.

La glycoprotéine inactivant les ribosomes dont les thiols sont bloqués est alors soumise à l'oxydation par les ions periodate. Si par contre la sous-unité cytotoxique ne présente pas de thiol, ou bien si le ou les thiols ne servent pas au couplage, le blocage indiqué ci-dessus n'est pas mis en oeuvre.

La réaction d'oxydation periodique est effectuée à un pH acide, compris entre 3 et 7, de préférence entre 5 et 6,5. Le periodate est utilisé en excès ; plus particulièrement, la concentration en periodate alcalin est supérieure à la concentration des diols vicinaux susceptibles d'être oxydés : des concentrations de 10 à 50 mM en periodate de sodium pour des concentrations de 1 à 10 mg/ml de sous-unité cytotoxique sont convenables. La durée du traitement, réalisé à une température comprise entre 0 et 15 ° C et de préférence entre 1 et 5 ° C et à l'obscurité,est comprise entre 0,2 et 24 h.

La réaction est arrêtée par addition d'un réactif qui consomme le periodate restant, par exemple un excès d'éthylèneglycol,et les sous-produits sont éliminés par dialyse. Le produit obtenu à la fin de la réaction est isolé selon les techniques conventionnelles.

Si les groupes thiol du produit de départ étaient bloqués, le déblocage est effectué selon les méthodes connues, par exemple par action d'un agent réducteur apte à libérer la fonction thiol préalablement bloquée tel que le mercapto-2 éthanol, ce qui conduit à l'obtention de la nouvelle glycoprotéine inactivant les ribosomes et à longue durée d'action prête à être utilisée pour le couplage avec un anticorps pour obtenir une immunotoxine.

Dans le cas de la chaîne A de ricine, la nouvelle molécule ainsi obtenue (symbolisée A-la) possède les propriétés principales suivantes :

- un poids moléculaire non significativement différent de celui de la chaîne A native. Ce procédé de modification ne fait apparaître de façon visible par électrophorèse en gradient de polyacrylamide des polymères de la protéine qu'en très faible quantité et pas de produits de dégradation,
- un taux de groupements thiol libres supérieur à 0,7 par mole,
- une immunoréactivité envers des anticorps de lapin anti-chaîne A de ricine que l'on ne peut distinguer de celle de la chaîne A native,
- une activité inhibitrice de la synthèse protéique dans un modèle acellulaire supérieure à 50 % de celle provoquée par une égale quantité de chaîne A native,
- enfin, après administration intraveineuse unique chez le lapin à une dose voisine de 0,4 mg/kg de poids corporel, le taux plasmatique de la chaîne A à longue durée d'action (A-la) présent dans le sang circulant au temps 23 h après l'injection est supérieur à 10 % du taux présent au temps zéro (contre 0,015 % pour la chaîne A native à ce même temps, soit un accroissement du taux plasmatique par un facteur largement supérieur à 500).

De même, dans le cas de la gélonine, la molécule obtenue par oxydation periodique, possède les propriétés principales suivantes:

- un poids moléculaire non significativement différent de celui de la gélonine native,
- une immunoréactivité envers les anticorps de lapin anti-gélonine que l'on ne peut distinguer de celle de la gélonine native,
- enfin, après administration intraveineuse unique chez le lapin à une dose voisine de 0,3 mg/kg de poids corporel, le taux plasmatique de la gélonine modifiée, au temps 24 h après l'injection, est supérieur à 3 % du taux présent au temps zéro (contre 0,01 % pour la gélonine native à ce même temps, soit un accroissement du taux plasmatique par un facteur supérieur à 200).

La préparation des conjugués ou immunotoxines à partir des glycoprotéines inactivant les ribosomes et à longue durée d'action est réalisée par n'importe quel procédé convenablement choisi dans la gamme de ceux qui sont décrits dans le brevet US 4 340 535. Si la sous-unité cytotoxique choisie présente naturellement au moins un thiol la rendant apte au couplage, cette fonction sera préférentiellement mise en oeuvre par réaction avec l'anticorps ou fragment d'anticorps porteur d'un groupement disulfure activé. Si la sousunité cytotoxique choisie ne possède pas naturellement de groupe thiol la rendant apte au couplage on pourra préférentiellement introduire artificiellement sur ladite sous-unité, après l'étape d'oxydation par les ions periodate, au moins un groupement fonctionnel porteur d'un thiol libre, selon tout procédé connu, et poursuivre le couplage comme indiqué ci-dessus.

L'introduction dudit groupement fonctionnel peut avoir lieu soit avant l'étape d'oxydation par les ions periodate mais il sera alors nécessaire que le radical thiol soit bloqué pendant l'étape d'oxydation puis débloqué après cette étape, soit après l'étape d'oxydation.

La préparation d'anticorps monoclonaux dirigés contre des cellules cancéreuses humaines a été largement mentionnée dans la littérature scientifique et beaucoup de ces anticorps sont maintenant disponibles commercialement.

Le couplage chimique entre la GPIR-la et l'anticorps (ou fragment d'anticorps) peut être réalisé selon le procédé de la présente invention par des modes opératoires qui :

- préservent les activités biologiques respectives des deux composants du conjugué : l'anticorps et la GPIR-la,
- assurent au procédé une reproductibilité satisfaisante et un bon rendement de couplage,
- permettent de maîtriser la valeur du rapport GPIR-la/anticorps dans le conjugué obtenu,
- conduisent à un produit stable et soluble dans l'eau.

Parmi les modes opératoires répondant à ces caractéristiques, il faut privilégier ceux qui mettent en oeuvre une ou plusieurs fonctions thiol pour l'établissement de la liaison entre les 2 protéines. En effet, ces fonctions thiol se prêtent particulièrement bien à l'établissement soit de liaisons disulfure, soit de liaisons thioéther qui satisfont les unes comme les autres aux conditions générales ci-dessus.

La préparation d'immunotoxines ayant en même temps les caractéristiques suivantes :

- la liaison covalente entre la chaîne A de la ricine et l'anticorps contient un radical disulfure,
- l'un des atomes de soufre constituant la liaison disulfure est toujours l'atome de soufre appartenant au résidu de cystéine en position 257 de la chaîne A de ricine,
- le bras de liaison réunissant la chaîne A de ricine et l'anticorps est fixé sur ce dernier au niveau de groupements $NH_2$ latéraux ou terminaux d'une chaîne peptidique,

et formées par couplage d'un anticorps avec la chaîne A de la ricine est décrite en détail dans le brevet US 4 340 535.

La même méthode peut être appliquée à la préparation d'immunotoxines ayant les mêmes caractéristiques et formées par couplage d'un anticorps ou fragment d'anticorps avec une GPIR-la.

La préparation d'immunotoxines formées par couplage d'un anticorps ou fragment d'anticorps avec une GPIR-la et par une liaison covalente du type disulfure ou thioéther au niveau de différents groupes fonctionnels est décrite en détail ci-après.

D'une façon générale, pour la bonne conduite des réactions de couplage entre protéines et en vue d'éliminer notamment les réticulations anarchiques, il importe que l'une des protéines à coupler et elle seule porte la ou les fonctions thiol à mettre en oeuvre, alors que l'autre protéine et elle seule porte une ou plusieurs fonctions susceptibles de réagir avec les thiols en milieu aqueux de pH compris entre 5 et 9 et à une température ne dépassant pas 30 ° C, pour fournir une liaison covalente stable et définie.

Les caractéristiques des protéines $P_1$ et $P_2$ utilisées comme produits de départ sont illustrées en détail ci-après. La structure d'espacement E peut être remplacée par les structures préférentielles R à $R_8$ qui ne sont données qu'à titre d'exemple.

## I - CAS DE LA PROTEINE $P_1$

Cette protéine étant dans tous les cas celle qui porte la ou les fonctions thiol qui participeront au couplage, la situation se présente de façon diverse selon la nature de cette protéine $P_1$.

A) La protéine $P_1$ porte naturellement un ou plusieurs radicaux thiol utilisables pour permettre le couplage à la protéine $P_2$ : c'est notamment le cas si la protéine $P_1$ est le fragment d'anticorps connu sous la dénomination de F(ab)' , tel qu'il est classiquement obtenu par protéolyse limitée de l'anticorps en présence de pepsine, suivie d'une réduction du (ou des) pont(s) disulfure entre chaînes lourdes.

C'est également le cas si la protéine $P_1$ est une GPIR-la, par exemple la chaîne A de la ricine modifiée (A-la), ou un dérivé de cette dernière,où l'un au moins des groupements thiol portés par les résidus de cystéine 171 et 257 de la chaîne A de ricine native est libre et accessible au couplage chimique.

Dans tous ces cas, la protéine $P_1$ porteuse de son (ou ses) groupement(s) thiol naturel(s) peut être utilisée dans cet état pour l'étape de couplage.

B) La protéine $P_1$ ne porte naturellement pas de radicaux thiol utilisables pour permettre le couplage à la protéine $P_2$ :

- c'est notamment le cas si la protéine $P_1$ est une immunoglobuline native, un anticorps entier ou un fragment d'anticorps et notamment l'un des fragments conventionnellement dénommés $F(ab)'_2$ ou F(ab), - un autre cas où la protéine $P_1$ ne porte pas naturellement de fonction thiol utilisable pour le couplage est celui où cette protéine $P_1$ est une GPIR-la, par exemple la chaîne A de ricine à longue durée d'action où chacun des deux résidus de cystéine est soit bloqué par alkylation, soit inaccessible à la modification chimique.

Dans tous les cas, il conviendra alors d'introduire artificiellement sur de telles molécules une ou plusieurs fonctions thiol aptes à permettre le couplage.

Trois types de réaction peuvent être utilisés de préférence pour l'introduction de fonctions thiol :

1- Le premier type de réaction est l'action de l'anhydride S-acétylmercapto succinique qui est capable d'acyler des fonctions aminées de la protéine. On pourra ensuite libérer les fonctions thiol par élimination du radical acétyl protecteur, par action de l'hydroxylamine, ainsi que cela a été décrit (Archives of Biochemistry and Biophysics, 119, 41-49, 1967). On pourra même, dans le cas où la (ou les) fonction(s) thiol ainsi introduites sous forme protégée doivent réagir ultérieurement avec un radical disulfure mixte activé, se dispenser de la déprotection préalable par l'hydroxylamine; en effet, la réaction de formation de la liaison disulfure utilisant les réactifs objets de la présente invention se produit aussi bien avec le radical S-acétyl qu'avec le thiol libre. D'autres méthodes décrites dans la littérature scientifique peuvent aussi être utilisées pour l'introduction de fonctions thiol sur la protéine à modifier.

2- Le deuxième type de réaction consiste à faire réagir la protéine par ses groupements carboxyliques avec une molécule diaminée symétrique présentant un pont disulfure, de formule :

$$H_2N-R_1-S-S-R_1-NH_2$$

dans laquelle $R_1$ est un groupe aliphatique comportant de 2 à 5 atomes de carbone.

La réaction se fait de préférence avec la cystamine/$R_1$ = $-(CH_2)_2-$/ en présence d'un agent de couplage tel qu'un carbodiimide et notamment un dérivé soluble dans l'eau comme l'éthyl-1 (diméthylamino-3 propyl)-3 carbodiimide, et conduit à la formation, selon les stoechiométries mises en oeuvre, de l'un des dérivés suivants ou du mélange des deux :

$$P_{1'}-CO-NH-R_1-S-S-R_1-NH_2 \quad (Ia)$$

$P_{1'}$-CO-NH-$R_1$-S-S-$R_1$-NH-CO-$P_1$ (Ib).

Un tel produit de réaction peut être alors utilisé de deux manières:

a) Si, dans les formules Ia ou Ib, la protéine $P_1$ est une GPIR-la, par exemple la chaîne A de ricine à longue durée d'action ou l'un de ses dérivés,le milieu réactionnel obtenu est soumis sans fractionnement à l'action d'un réducteur tel que le mercapto-2-éthanol, ce qui conduit à l'obtention d'un dérivé protéique unique de formule générale :

$P_{1'}$-CONH-$R_1$-SH

Le produit ainsi obtenu est alors purifié par dialyse ou filtration sur gel.

b) Si, dans les formules Ia et Ib, la protéine $P_1$ est un anticorps ou l'un de ses fragments, le milieu réactionnel obtenu sera utilisé tel quel pour le couplage en utilisant alors une méthode d'échange thiol/disulfure par exemple celle décrite par Gilliland et Collier (Cancer Research, 40, 3564, 1980).

3- Le troisième type de réaction consiste à utiliser des motifs glucidiques naturellement présents sur les anticorps pour fixer le radical porteur du thiol que l'on se propose d'introduire. La protéine P est alors soumise à une oxydation par les ions periodate selon les méthodes connues afin de faire apparaître des fonctions aldéhyde sur les motifs glucidiques. Après arrêt de la réaction par addition d'un excès d'éthylèneglycol et élimination des sous-produits et excès de réactifs par dialyse, le produit obtenu est traité par une molécule diaminée symétrique présentant un pont disulfure, de formule générale :

$H_2N$-$R_1$-S-S-$R_1$-$NH_2$

dans laquelle $R_1$ est un groupe aliphatique comportant de 2 à 5 atomes de carbone. Les produits d'addition formés sont alors réduits en amines secondaires ou tertiaires par action d'un hydrure métallique convenable, notamment le borohydrure de sodium. La réaction se fait de préférence avec la cystamine /$R_1$ = $-(CH_2)_2-$/ et conduit à la formation, selon les stoechiométries mises en oeuvre de l'un des dérivés suivants ou du mélange des deux :

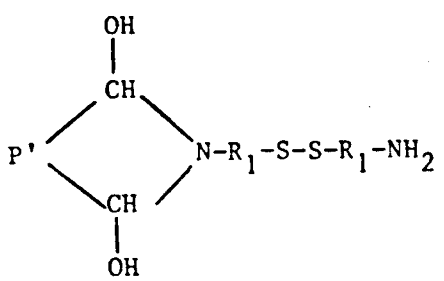

IIa

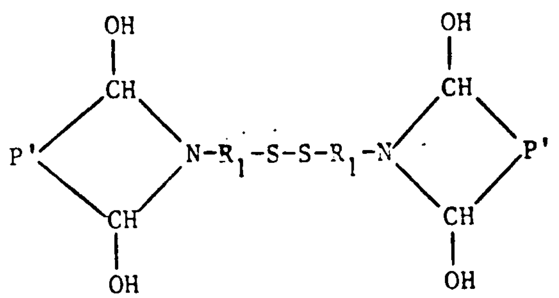

IIb

Le milieu réactionnel obtenu pourra alors être traité exactement comme indiqué ci-dessus pour les produits caractérisés par les structures Ia ou Ib.

Dans les deux derniers types de réaction d'introduction artificielle de groupements thiol décrits ci-dessus (ceux utilisant un réactif disulfure diaminé symétrique), il est préférable que la protéine $P_1$ mise en oeuvre ne possède ni groupes SH libres, ni groupes aminés libres.

Dans le cas de la GPIR-la cela peut toujours être réalisé par alkylation du ou des SH naturels obtenue par réaction avec un réactif usuel des thiols tel que le N-éthylmaléiimide ou l'acide iodacétique ou l'un de ses dérivés et par méthylation des $NH_2$ naturels selon le procédé de méthylation réductive décrit par MEANS et FEENEY (Biochemistry 7, 2192 (1968)). Par exemple, la chaîne A de ricine native modifiée peut présenter jusqu'à 6 radicaux méthyle par mole préalablement introduits. Une telle protéine conserve l'intégralité de ses propriétés biologiques et notamment sa capacité à inhiber la synthèse protéique ribosomale dans les cellules eucaryotes.

Dans les cas des anticorps ou fragments d'anticorps et plus généralement de toutes les substances du premier groupe tel que défini précédemment qui ne possèdent pas de groupements SH naturellement libres, il conviendra de procéder à une méthylation réductive, par exemple selon la méthode de MEANS et FEENEY : on peut ainsi habituellement introduire plusieurs dizaines de radicaux méthyle par mole d'anticorps sans modifier sa capacité à reconnaître sélectivement un antigène à la surface des cellules porteuses de cet antigène.

## II - CAS DE LA PROTEINE $P_2$

Cette protéine est dans tous les cas celle qui porte un ou plusieurs groupements fonctionnels capables de réagir avec les thiols de la protéine $P_1$ pour former une liaison soit disulfure soit thioéther. Ces groupements fonctionnels, toujours artificiellement introduits sur la protéine $P_2$, diffèrent selon que l'on cherche à obtenir un couplage par liaison disulfure ou par liaison thioéther et sont choisis comme indiqué ci-après.

### 1) Cas de la liaison disulfure

La préparation du conjugué peut alors être représentée par le schéma :

$$P_{1'}\text{-(Z-Y-E)}_n\text{-SH} + P_{2'}\text{-Z'-Y'-E'-S-S-X} \rightarrow P_{1'}\text{-(Z-Y-E)}_n\text{-S-S-E'-Y'-Z'-}P_{2'} + \text{X-SH}$$

La protéine $P_2$ substituée par un atome de soufre activé est obtenue à partir de la protéine $P_2$ elle-même ou de la protéine $P_2$ correctement protégée, par substitution à l'aide d'un réactif lui-même porteur d'un atome de soufre activé selon le schéma :

$$P_2 + \text{L-Y'-R-S-S-X} \rightarrow P_{2'}\text{-Z'-Y'-R'-S-S-X}$$

dans lequel :

- $P_2$ désigne la protéine à substituer
- L-Y' représente une fonction permettant la fixation covalente du réactif sur la protéine.

Le groupement fonctionnel L-Y' est une fonction capable de se lier de façon covalente avec l'une quelconque des fonctions portées par les chaînes latérales des aminoacides constitutifs de la protéine à substituer. Parmi celles-ci, on retiendra particulièrement :

a) Les fonctions aminées terminales des chaînes peptidiques ou les fonctions aminées latérales des radicaux lysyle contenus dans la protéine. Dans ce cas, L-Y' pourra notamment représenter :

- un groupe carboxylique qui pourra se lier aux fonctions aminées de la protéine en présence d'un agent de couplage tel qu'un carbodiimide et notamment un dérivé soluble dans l'eau comme l'éthyl-1 (diméthylamino-3 propyl)-3 carbodiimide
- un chlorure d'acide carboxylique qui est susceptible de réagir directement avec les fonctions aminées pour les acyler
- un ester dit "activé" tel qu'un ester d'ortho- ou para-, nitro- ou dinitro-phényle ou encore un ester de N-hydroxy succinimide qui peut réagir directement avec les fonctions aminées pour les acyler
- un anhydride interne d'un diacide carboxylique tel, par exemple, l'anhydrique succinique qui réagit spontanément avec les fonctions amines pour créer des liaisons amides

- un groupement imidoester

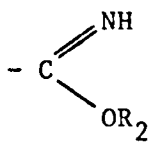

(L = $OR_2$, Y' = -(C=NH)-)

où R est un groupe alkyle, réagissant avec les groupes aminés de la protéine $P_2$ selon la réaction :

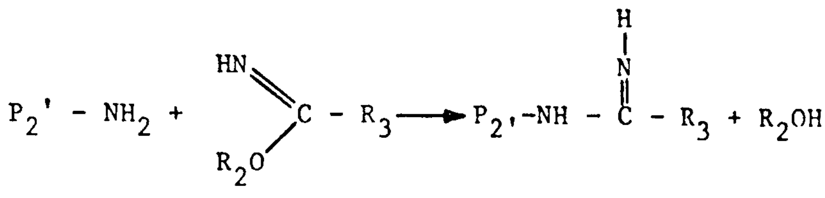

où $R_3$ représente le groupe -R-S-SX ;

b) les fonctions phénol des radicaux tyrosyle contenus dans la protéine. Dans ce cas L-Y' pourra notamment représenter un groupement imidazolyl-1-carbonyle, réagissant avec les groupes phénol de la protéine selon la réaction :

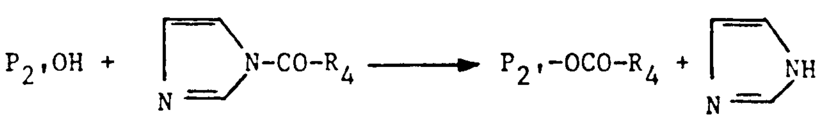

où le 1-imidazolyle est L, le groupe CO est Y' et $R_4$ est le groupe -R-S-S-X.

Le radical -S-S-X désigne un disulfure mixte activé capable de réagir avec un radical thiol libre. En particulier, dans ce disulfure mixte, X pourra désigner un groupe pyridyl-2 ou pyridyl-4, éventuellement substitué par un ou des radicaux alkyle, halogène ou carboxylique. X peut aussi désigner un groupe phényle de préférence substitué par un ou des groupes nitro- ou carboxylique. X peut encore représenter un groupe alcoxycarbonyle tel que le groupe méthoxycarbonyle.

Le radical R désigne la structure d'espacement (indiquée comme E dans la formule générale II ci-dessus) capable de porter simultanément les substituants Y' et S - S - X. Il devra être choisi de façon à ne pas comporter de fonctions susceptibles d'interférer au cours des réactions ultérieures avec les réactifs utilisés et les produits synthétisés. En particulier, le groupe R peut être un groupe $-(CH_2)_n-$ avec n compris entre 1 et 10, ou encore un groupe :

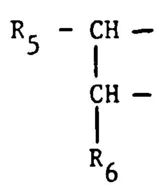

dans lequel $R_6$ désigne l'hydrogène ou un groupe alkyle ayant de 1 à 8 atomes de carbone et $R_5$ désigne un substituant inerte vis-à-vis des réactifs utilisés ultérieurement tel qu'un groupe carbamate

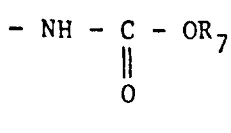

où $R_7$ désigne un groupe alkyle droit ou ramifié ayant de 1 à 5 atomes de carbone et notamment le

groupe tertiobutyle.

La réaction du composé L-Y'-R-S-S-X avec la protéine $P_2$ est effectuée en phase liquide homogène le plus souvent dans l'eau ou une solution tampon. Lorsque la solubilité des réactifs l'exige, il est possible d'ajouter au milieu réactionnel un solvant organique miscible à l'eau à une concentration finale pouvant atteindre 20 % en volume lorsqu'il s'agit d'un alcool tertiaire tel que le butanol tertiaire ou 10 % en volume lorsqu'il s'agit du diméthylformamide ou du tétrahydrofuranne.

La réaction est effectuée à température ambiante pendant un temps variant de quelques minutes à quelques heures. Après quoi, une dialyse ou une filtration sur gel permet d'éliminer les produits de faible masse moléculaire et, en particulier, les excès de réactifs. Ce procédé permet d'introduire un nombre de groupements substituants par mole de protéine habituellement compris entre 1 et 15.

En utilisant de tels composés, le couplage avec la protéine $P_1$ est réalisé par mise en présence des deux protéines en solution aqueuse de pH compris entre 6 et 8, à une température ne dépassant pas 30 ° C, pendant un temps variant de 1 h à 24 h. La solution aqueuse obtenue est éventuellement dialysée pour éliminer les produits de faible masse moléculaire, puis le conjugué peut être purifié par diverses méthodes connues.

2) Cas de la liaison thioéther

La préparation du conjugué consiste alors à faire réagir $P_{1'}$-(Z-Y-E)$_n$-SH avec la protéine $P_2$ sur laquelle auront préalablement été introduits un ou plusieurs radicaux maléiimide.

La réaction est alors représentée par le schéma suivant cité comme exemple :

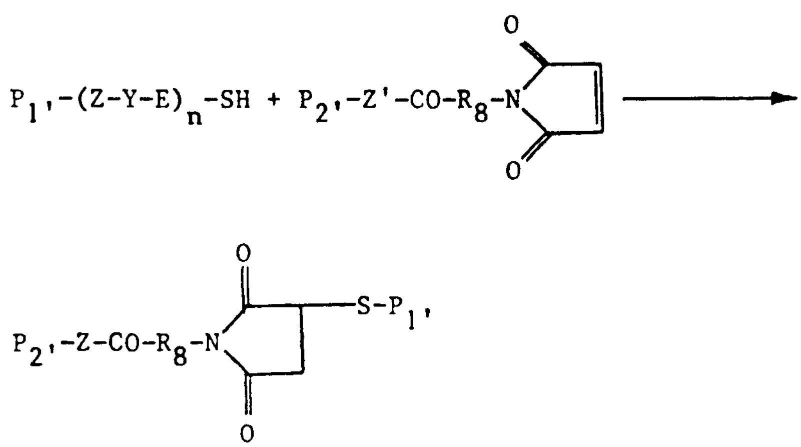

dans lequel :

- $R_8$ représente une structure d'espacement, aliphatique ou aromatique, comportant de 1 à 15 atomes de carbone, inerte vis-à-vis des réactifs utilisés ultérieurement
- Z représente des groupes variables selon le type de groupement fonctionnel substitué sur la protéine $P_2$.

Ainsi

Z = oxygène dans le cas d'un ester sur le phénol d'un résidu tyrosyle

Z = NH dans le cas du couplage d'un groupement carboxylique activé sur un groupement aminé de la protéine

Z = $NH-CH_2$ dans le cas de la réaction d'une chlorométhylcétone sur un groupement aminé de la protéine.

La protéine $P_2$ substituée par le ou les groupes maléiimide est obtenue à partir de la protéine $P_2$ elle-même ou de la protéine $P_2$ correctement protégée, par substitution de fonctions convenables de la protéine à l'aide d'un réactif lui-même porteur du groupement maléiimide. Parmi ces fonctions convenables, on retiendra particulièrement :

a) Les fonctions aminées terminales des chaînes peptidiques ou les fonctions aminées latérales des résidus lysyle contenus dans la protéine. Dans ce cas, le réactif porteur du radical maléiimide pourra être :

- soit un réactif de formule générale

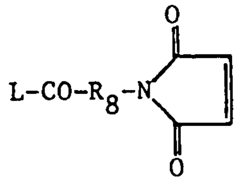

dans laquelle L-CO- représente :
- soit un groupe carboxylique, la réaction étant alors effectuée après activation de la fonction carboxylique en présence d'un agent de couplage tel qu'un carbodiimide et notamment un dérivé soluble dans l'eau tel que l'éthyl-1 (diméthylamino-3 propyl)-3 carbodiimide,
- soit un ester dit "activé" tel qu'un ester d'ortho- ou para- , nitro ou dinitrophényle, ou encore un ester de N-hydroxysuccinimide qui réagit directement avec les fonctions aminées pour les acyler.

La préparation de tels réactifs est notamment décrite dans Helvetica Chimica Acta 58, 531-541 (1975). D'autres réactifs de la même classe sont commercialement disponibles.

- soit un réactif de formule générale :

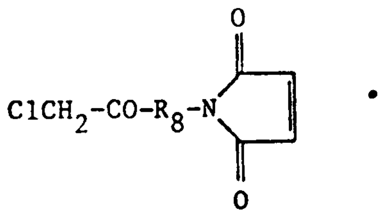

capable de réagir avec les groupements aminés de la protéine $P_2$ selon le schéma :

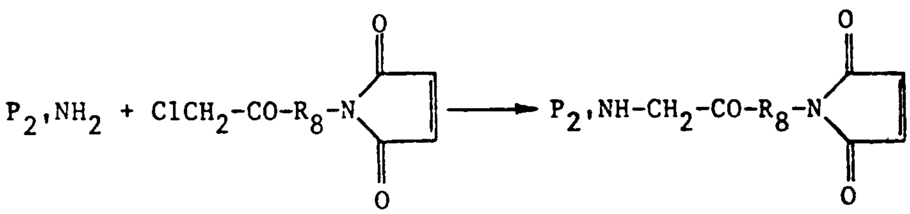

b) Les fonctions phénol des radicaux tyrosyle contenus dans la protéine. Dans ce cas, le réactif porteur du radical maléiimide pourra être un réactif de formule générale :

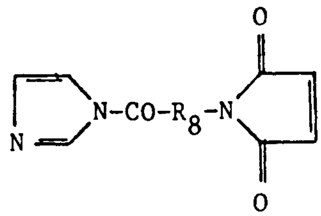

réagissant avec les groupes phénol de la protéine selon la réaction :

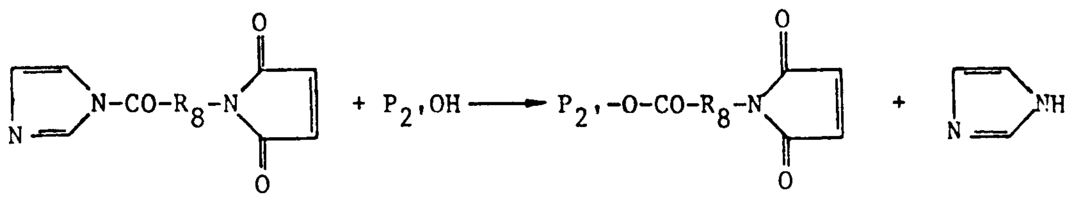

La réaction des réactifs porteurs de maléiimide avec la protéine $P_2$ est effectuée en phase liquide homogène le plus souvent dans l'eau ou une solution tampon. Lorsque la solubilité des réactifs l'exige, il est possible d'ajouter au milieu réactionnel un solvant organique miscible à l'eau à une concentration finale pouvant atteindre 20 % en volume lorsqu'il s'agit d'un alcool tertiaire tel que le butanol tertiaire ou 10 % en volume lorsqu'il s'agit du diméthylformamide ou du tétrahydrofuranne.

La réaction est effectuée à température ambiante pendant un temps variant de quelques minutes à quelques heures. Après quoi, une dialyse ou une filtration sur gel permet d'éliminer les produits de faible masse moléculaire et, en particulier, les excès de réactifs. Ce procédé permet d'introduire un nombre de groupements substituants par mole de protéine habituellement compris entre 1 et 15.

En utilisant de tels composés, le couplage avec la protéine $P_1$ est réalisé par mise en présence des deux protéines en solution aqueuse de pH compris entre 6 et 8, à une température ne dépassant pas 30 ° C, pendant un temps variant de 1 h à 24 h. La solution obtenue est éventuellement dialysée pour éliminer les produits de faible masse moléculaire, puis le conjugué peut être purifié par diverses méthodes connues.

Les composés de formule

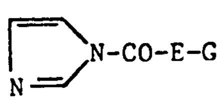

VI

dans laquelle E et G sont tels que définis ci-dessus, sont préparés par un procédé caractérisé en ce qu'on fait réagir un composé de formule

G-E-COOH VII

dans laquelle G et E sont tels que définis ci-dessus, avec le carbonyldiimidazole de formule

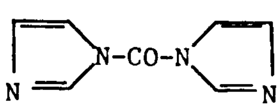

VIII

dans un solvant organique à la température de 10 à 40 ° C.

Les composés de formule VI sont particulièrement utiles comme agents de couplage sur les hydroxyles des tyrosines des protéines GPIR-Ia et P.

Selon un autre de ses aspects, la présente invention a pour objet de nouveaux produits ayant la formule statistique suivante

GPIR-la"-O-CO-E-G     IX

dans laquelle

- GPIR-la" représente le radical de la protéine GPIR-la ou toute molécule dérivant de ladite GPIR-la par modification artificielle de l'un quelconque de ses groupements fonctionnels, privée d'un ou plusieurs groupes hydroxyles phénoliques des tyrosines
- l'atome d'oxygène est celui des groupes hydroxyles phénoliques manquant dans le radical GPIR-la" ;
- E et G sont tels que définis ci-dessus.

Particulièrement préférés sont les composés de formule IX dans laquelle E représente un groupe $-(CH_2)_p-$ où p est un nombre entier de 2 à 7 ou un groupe

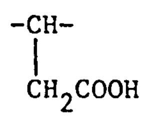

et G est un groupement de structure -S-S-X, où X est un radical activateur choisi parmi les groupes 2-pyridyle et 4-pyridyle non substitués ou substitués par un ou des halogènes ou des radicaux alkyle, carboxyle, alcoxycarbonyle, le groupe phényle non substitué ou substitué par un ou des halogènes ou des groupes nitro, alcoxy, carboxyle ou alcoxycarboxyle, ou un groupe alcoxycarbonyle.

Les produits de formule IX sont préparés en faisant réagir un produit de formule

GPIR-la"-OH

dans laquelle GPIR-la" est tel que défini ci-dessus et le groupe hydroxyle est l'hydroxyle phénolique manquant dans les tyrosines du radical GPIR-la", avec un composé de formule VI ci-dessus à une température de 10 à 40 ° C dans un solvant aqueux contenant éventuellement un solvant organique miscible à l'eau, tel que par exemple un solvant éthéré comme le dioxanne ou le tétrahydrofuranne.

Dans le cas où GPIR-la est la chaîne A de ricine à longue durée d'action, les propriétés des immunotoxines IT (A-la) ainsi obtenues sont les suivantes :

- le taux de couplage moyen exprimé en nombre de moles de chaîne A modifiée par mole d'anticorps est habituellement compris entre 0,5 et 5 et en particulier entre 1 et 3,
- la séparation électrophorétique en gel de polyacrylamide de l'IT (A-la) conduit à une répartition du produit en une série de bandes correspondant à des produits dont les poids moléculaires diffèrent de celui de l'anticorps par incréments successifs de 30 000 daltons,
- les études effectuées par cytofluorométrie permettent de montrer que l'anticorps n'a subi aucune altération importante au cours des réactions d'activation et de couplage auxquelles il a été soumis et qu'il reste capable au sein même du conjugué de reconnaître l'antigène contre lequel il est dirigé,
- l'activité inhibitrice de la synthèse protéique de la chaîne A, modifiée et couplée à un anticorps, déterminée dans un modèle acellulaire en présence de mercapto-2-éthanol est totalement conservée.

L'activité cytotoxique des IT (A-la) mesurée en présence d'activateur dans un test de synthèse protéique en modèle cellulaire sur les cellules présentant l'antigène-cible est plus de 1000 fois supérieure à celle mesurée dans les mêmes conditions sur des cellules ne présentant pas l'antigène-cible. Par exemple, l'immunotoxine (désignée IT (A-la) T101), construite en couplant à l'aide d'un bras à pont disulfure la chaîne A de ricine modifiée à un anticorps monoclonal (désigné anticorps T101) dirigé contre l'antigène T65 présent à la surface de certaines cellules leucémiques humaines, est environ $10^5$ fois plus cytotoxique vis-à-vis des cellules T65 positives que des cellules T65 négatives,

- l'efficacité cytotoxique des IT (A-la) mesurée en test clonogénique est aussi élevée que celle obtenue avec les IT conventionnelles correspondantes. Par exemple l'IT (A-la) T101 à une dose aussi faible que $10^{-11}$ M, en présence de 10 mM de chlorure d'ammonium,conduit à une cytoréduction spécifique de l'ordre de 99,999 % de la valeur initiale. Ce résultat est identique à ceux obtenus avec l'IT-T101, construite avec le même anticorps et la chaîne A de ricine non modifiée,

- enfin, après administration intraveineuse de l'IT (A-la) chez le lapin à une dose voisine de 0,4 mg/kg de poids corporel exprimé en chaîne A, le taux plasmatique de l'IT (A-la) présent dans le sang circulant au temps 23 h après l'injection est de 10 à 200 fois supérieur au taux plasmatique de l'IT conventionnelle mesurée dans les mêmes conditions. Ainsi typiquement chez le lapin il apparaît que le taux plasmatique de l'IT (A-la) T101 dans le sang circulant au temps 23 h après l'injection est de 7 % du produit présent au temps zéro contre 0,05 % pour l'IT T101 conventionnelle correspondante, au même temps, soit un accroissement de l'ordre d'un facteur 140.

On obtient ainsi des immunotoxines modifiées qui ont acquis un caractère nouveau quant à leurs propriétés pharmacocinétiques.

Plus particulièrement, par modification appropriée de la sous-unité cytotoxique, on a pu ajouter aux propriétés de cytotoxicité spécifique des immunotoxines, sans qu'il leur soit porté atteinte, une nouvelle propriété tout aussi intrinsèque : la capacité à manifester une lente cinétique d'élimination plasmatique.

Les exemples suivants permettent de mieux comprendre l'invention sans en limiter la portée.

## Exemple 1

Cet exemple démontre, après injection intraveineuse chez l'animal, l'élimination lente de la chaîne A de ricine modifiée par le periodate de sodium.

### Modification de la chaîne A de ricine par le periodate de sodium.

1) Blocage du SH naturel par le DTNB.

La chaîne A de ricine a été préparée et purifiée ainsi qu'il a été indiqué dans le brevet US 4 340 535. A 10 ml d'une solution de chaîne A de ricine à 5,6 mg/ml (à 0,84 groupement thiol par chaîne A) dans le tampon PBS (tampon phosphate 20 mM, NaCl 150 mM, pH 7) on ajoute 20 équivalents d'une solution d'acide dinitro-2,2' dithio-5,5' dibenzoïque (DTNB) soit 385 microlitres d'une solution 0,1 M de DTNB dans un tampon phosphate 125 mM pH 7 (cette solution est amenée à pH 7 à l'aide d'hydroxyde de sodium). On laisse l'incubation se poursuivre pendant 20 min à 20 °C. On dialyse ensuite la solution contre du tampon PBS à 4 °C, on obtient ainsi 53 mg de chaîne A bloquée sur la fonction thiol, en solution à 5 mg/ml.

2) Oxydation périodique de la chaîne A bloquée :

A 6 ml de solution à 5 mg/ml de chaîne A bloquée, amenée à pH 6 à l'aide d'acide acétique 1 M, on ajoute 120 microlitres d'une solution de periodate de sodium 0,5 M dans l'eau. On laisse l'incubation se poursuivre pendant 16 h à 4 °C dans l'obscurité. La réaction d'oxydation est arrêtée par addition de 620 microlitres d'une solution aqueuse d'éthylèneglycol 1 M. Après 15 min d'incubation à 20 °C, le milieu réactionnel est dialysé à 4 °C contre du tampon PBS. L'oxydation periodique fait apparaître un léger précipité protéique qui est éliminé par centrifugation à 10 000 x g pendant 30 min. On obtient ainsi 24 mg de chaîne A bloquée et oxydée à une concentration de 3,4 mg/ml.

3) Déblocage des groupements thiol :

A 6 ml de chaîne A bloquée et oxydée à 3,4 mg/ml dans le tampon A on ajoute comme agent réducteur du mercapto-2 éthanol à la concentration finale de 1 %. On laisse l'incubation se poursuivre pendant 1 h à 20 °C. On dialyse ensuite la solution contre le tampon PBS à 4 °C. On obtient ainsi 19 mg de chaîne A oxydée à une concentration de 2,8 mg/ml.

En utilisant la technique au DTNB (Methods in Enzymology, 1972, 25 457 (Academic Press) on détermine que la chaîne A modifiée obtenue présente 0,70 groupement thiol libre par mole. Le poids moléculaire de la chaîne A modifiée est de 30 000 +/- 3000, déterminé par électrophorèse en gradient de polyacrylamide en présence de dodécyl-sulfate de sodium.

La préparation de chaîne A dont les motifs polysaccharidiques ont été oxydés, obtenue précédemment, a été étudiée en ce qui concerne ses activités enzymatiques d'inhibition de la synthèse protéique et ses propriétés pharmacocinétiques.

### II- Activité enzymatique de la chaîne A à longue durée d'action, mesurée sur un modèle acellulaire.

La propriété biologique fondamentale de la chaîne A de ricine est d'inhiber la synthèse protéique cellulaire par altération de la sous-unité ribosomale 60S.

Le protocole in vitro utilisé comporte l'emploi de fractions subcellulaires de foie de rat convenablement complémentées et capables d'incorporer la 14C-phénylalanine en présence d'un ARN messager artificiel, l'acide polyuridylique.

Le mode opératoire employé pour préparer les fractions subcellulaires et pour mesurer l'incorporation de 14C-phénylalanine est une adaptation de la méthode décrite dans Biochemica Biophysica Acta, 1973, 312, 608-615, mettant en oeuvre à la fois une fraction microsomale et une fraction de cytosol des hépatocytes de rat. L'échantillon contenant la chaîne A est introduit sous la forme d'une solution convenablement diluée dans un tampon Tris HCl 50 mM, pH 7,6 contenant du mercapto-2 éthanol à 0,2 % et de la sérum-albumine bovine à 15 microgrammes/ml.

A partir des données de comptage, on calcule par rapport à un milieu témoin sans inhibiteur le pourcentage d'inhibition de l'incorporation de 14C-phénylalanine dans les protéines pour chaque milieu réactionnel contenant de la chaîne A de ricine.

L'activité inhibitrice a été déterminée. On observe une $CI_{50}$ égale à $7,8.10^{-9}$ mole/l pour la chaîne A oxydée. La $CI_{50}$ de la chaîne A témoin de l'expérience est de $13.10^{-9}$ mole/l : la modification n'entraîne donc pas de perte d'activité de la chaîne A.

III- Propriétés pharmacocinétiques de la chaîne A à longue durée d'action modifiée sur ses motifs polysaccharidiques.

La chaîne A est administrée chez le lapin par injection unique dans une veine de l'oreille. La quantité de chaîne A injectée correspond à 0,415 mg/kg. Les échantillons de sang sont prélevés au cours du temps sur héparine. Les plasmas sont analysés à l'aide d'un test immunoradiométrique ci-après désigné par l'abréviation IRM-I.

Cette technique a l'avantage de doser la chaîne A sans modification de celle-ci. Ce dosage est réalisé dans des plaques à microtitrations (par exemple : "NUNC-TSP screening system" Poly Labo Block France) dont le couvercle est muni de pointes hyperabsorbantes qui plongent dans les puits du socle. Ces pointes constituent les phases solides. Des anticorps de brebis anti-chaîne A de ricine (ci-après désignés sous l'abréviation AcI), purifiés par chromatographie d'affinité, sont absorbés sur les phases solides. Pour cela 200 microlitres d'une solution d'AcI à 10 microgrammes/ml dans le tampon phosphate PBS sont distribués dans les puits. Les pointes sont mises en contact d'abord avec la solution d'AcI pendant 24 h à 4 ° C puis avec du sérum de veau foetal pendant 3 h à 20 ° C pour saturer tous les sites de fixation. L'immunoabsorbant saturé est alors mis en contact pendant 3 h à 20 ° C avec les échantillons plasmatiques à doser à différentes dilutions ou avec des solutions de chaîne A de concentrations connues pour l'établissement de la courbe d'étalonnage. Un lavage est effectué par un tampon PBS, puis l'immunoabsorbant est mis en contact pendant 2 h à 20 ° C avec les anticorps de brebis anti-chaîne A de ricine purifiés par chromatographie d'affinité et radiomarqués (ci-après désignés par l'abréviation Ac2). Le radiomarquage de l'Ac2 est réalisé avec l'iode 125 en présence de chloramine T selon la méthode de Greenwood et Hunter (Biochem. J., 1963, 89, 114) ; l'activité spécifique des Ac2 radiomarqués est de 5 à 10 microCuries/microgramme. $10^6$ cpm d'Ac2 radiomarqués sont introduits sous 200 microlitres dans le tampon PBS, contenant 0,1 % de sérum-albumine bovine. Après lavage, dans le tampon PBS, les pointes sont détachées et la quantité d'Ac2 lié est mesurée par comptage de la radioactivité. La mesure de la concentration en chaîne A dans les échantillons à doser se fait par référence à la courbe d'étalonnage réalisée avec la chaîne A introduite à différentes concentrations connues. Lorsque de la chaîne A à longue durée d'action est injectée chez l'animal, cette même chaîne A à longue durée d'action est utilisée pour l'établissement de la courbe d'étalonnage correspondante.

Les valeurs de concentration en chaîne A dans le plasma sanguin mesuré par cette technique sont reproductibles et fiables. Le seuil de détection est de 1 nanogramme/ml. L'étude de la reproductibilité intra- et inter-essais donne des coefficients de variation inférieurs à 10 % pour les valeurs de concentration comprises dans la gamme de 1 à 200 nanogrammes/ml.

Les résultats de ces expériences sont représentés sous la forme de courbes présentant en abscisse le temps, exprimé en heure, et en ordonnée, sur échelle logarithmique, la concentration plasmatique du produit mesuré, rapportée en pourcent de la concentration plasmatique théorique au temps zéro. Cette valeur appelée "concentration plasmatique relative" (CPR) est calculée à l'aide de l'expression suivante :

$$CPR = \frac{\text{Concentration mesurée au temps t}}{\text{quantité injectée/volume plasmatique}} \times 100$$

Le volume plasmatique est considéré égal à 36 ml/kg de poids corporel de l'animal.

La figure 1 montre la courbe d'élimination plasmatique, en fonction du temps, de la chaîne A de ricine native injectée par voie intraveineuse. Cette courbe (courbe 1) présente deux phases : dans la première phase, le produit disparaît très rapidement de la circulation puisque trois heures après l'injection il ne reste plus dans le plasma que 0,1 % de la dose administrée. Dans la deuxième phase, la décroissance est plus lente.

Lorsque la chaîne A a été oxydée sur ses motifs polysaccharidiques, le profil d'élimination est profondément modifié : la première phase d'élimination - responsable de la disparition de la plus grande partie du produit - est pratiquement abolie, ce qui conduit à un accroissement considérable des taux plasmatiques de chaîne A. Vingt heures apres l'injection, la concentration de la chaîne A oxydée est 600 fois supérieure à ce qu'elle est quand la chaîne A n'est pas modifiée (courbe 2).

Exemple 2

Cet exemple démontre l'importance de la durée du traitement oxydatif sur les propriétés pharmacocinétiques de la chaîne A oxydée.

Six préparations de chaîne A oxydée sont réalisées en utilisant la procédure indiquée dans l'exemple 1 excepté pour la durée du traitement au periodate de sodium. Les temps de traitement sont les suivants : zéro : (arrêt immédiat par l'éthylèneglycol), 20 min, 40 min, 2,5 h, 4 h et 18 h.

Ces différentes préparations sont injectées chez le lapin et la concentration plasmatique relative de la chaîne A est mesurée au temps 23 h selon la même procédure que dans l'exemple 1.

Les résultats sont représentés sur la figure II. Ces résultats indiquent que 1) l'accroissement du taux plasmatique de la chaîne A est bien dû à l'oxydation periodique puisque lorsque la réaction est immédiatement arrêtée la concentration plasmatique de chaîne A est identique à celle obtenue pour la chaîne A native, 2) il est nécessaire que la durée de cette réaction soit relativement longue pour obtenir des effets optimaux.

Exemple 3

Cet exemple démontre, après injection intraveineuse chez l'animal, 1) l'élimination rapide de la gélonine native et 2) l'élimination lente de la gélonine modifiée par le periodate de sodium.

Modification de la gélonine par le periodate de sodium.

La gélonine a été préparée et purifiée à partir de Gelonium multiflorum selon la méthode décrite (J. Biol. Chem. (1980), 255, 6947-6953). La réaction d'oxydation est réalisée dans les mêmes conditions que celles décrites pour la chaîne A de ricine dans l'exemple 1 excepté que l'étape de blocage des thiols par le DTNB n'est pas pratiquée.

En effet, le couplage de la gélonine à l'anticorps n'étant pas généralement pratiqué à partir des groupements thiols naturels de la gélonine, les groupements thiols seront introduits artificiellement après l'étape d'oxydation, selon la technique décrite dans Cancer Res. 1984, 44, 129-133. A 1 ml de solution à 3 mg/ml de gélonine dans le tampon PBS, amenée à pH 6 à l'aide d'acide acétique 1M, on ajoute 21 microlitres d'une solution de periodate de sodium 0,5 M dans l'eau. On laisse l'incubation se poursuivre pendant 16 h à 4 °C dans l'obscurité. La réaction est arrêtée par addition de 105 microlitres d'une solution aqueuse d'éthylèneglycol 1 M. Après 15 min d'incubation à 20 °C, le milieu réactionnel est dialysé à 4 °C contre du tampon PBS. On obtient ainsi après centrifugation à 10 000 x g pendant 30 min 2,9 mg de gélonine oxydée à une concentration de 2,5 mg/ml.

Comme la chaîne A de ricine, la propriété fondamentale de la gélonine est d'inhiber la synthèse protéique de cellules eucaryotes par altération de la sous-unité ribosomale 60 S (Biochem. J., 1982, 207, 505-509). Dans le cas de la gélonine aussi, la modification due à l'oxydation periodique n'entraîne pas de perte d'activité.

II- Propriétés pharmacocinétiques de la gélonine à longue durée d'action.

La gélonine native ou modifiée selon les procédures développées ci-dessus est administrée chez le lapin par injection unique dans une veine de l'oreille. La quantité de gélonine injectée est comprise entre 0,3 et 0,4 mg/kg. Les échantillons de sang sont prélevés au cours du temps sur héparine. Les plasmas sont analysés à l'aide d'un test immunoradiométrique ci-après désigné par l'abréviation IRM-2.

Cet essai est réalisé selon la même technique que pour le test IRM-1 excepté que la solution Ac1 est ici une solution d'anticorps de lapin anti-gélonine purifiés par chromatographie d'affinité, les anticorps Ac2 étant les mêmes anticorps radiomarqués. La procédure de radiomarquage est identique à celle décrite pour la technique IRM-1. La mesure de la concentration en gélonine native ou gélonine modifiée dans les échantillons à doser se fait par référence à une courbe d'étalonnage réalisée avec la gélonine native ou modifiée introduite à différentes concentrations connues. L'essai IRM-2 répond aux mêmes caractéristiques de fiabilité et reproductibilité que celles décrites pour la technique IRM-1. Les résultats de ces expériences sont représentés de la même façon que pour la chaîne A de ricine dans l'exemple 1.

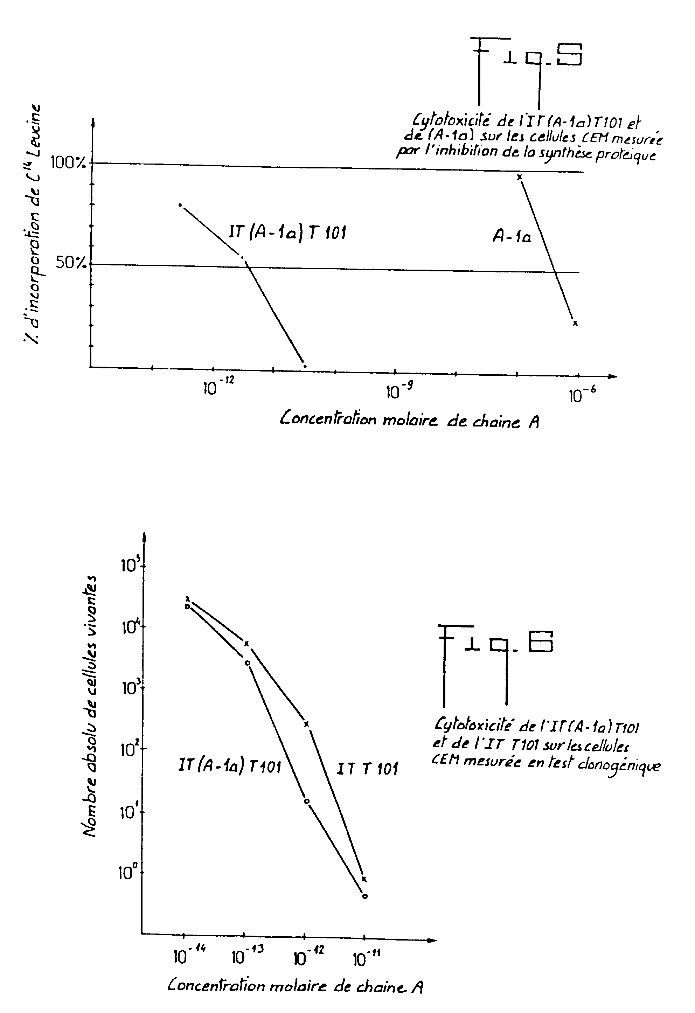

La figure 3 montre les courbes d'élimination plasmatique, en fonction du temps, de la gélonine native et de la gélonine oxydée injectées par voie intraveineuse. La gélonine native, comme la chaîne A de ricine native, disparaît très rapidement de la circulation puisque 99,99 % de la gélonine présente dans le sang circulant disparaissent en 24 h (courbe 1). Lorsque la gélonine a été oxydée sur ses motifs polysaccharidiques, le profil d'élimination est profondément modifié: 24 h après l'injection, la concentration de la gélonine oxydée est 300 fois supérieure à celle de la gélonine native (courbe 2).

Ainsi, comme pour la chaîne A de ricine, ces résultats prouvent que l'oxydation periodique a modifié les sucres impliqués dans le processus de reconnaissance responsable de l'élimination de la gélonine au point d'empêcher cette reconnaissance.

Exemple 4

Conjugué obtenu par réaction entre un anticorps anticellules T humaines (anticorps dirigé contre l'antigène T65) substitué par des groupements disulfure activé et la chaîne A de ricine oxydée.

a) Anticorps anticellules T humaines (ou anticorps T101) :

Cet anticorps a été obtenu selon la méthode décrite dans Journal of Immunology, 1980, 125 (2), 725-737.

b) Chaîne A de ricine oxydée :

La chaîne A de ricine a été préparée ainsi qu'il a été indiqué dans l'exemple 1.

II) Anticorps activés anticellules T humaines

A 100 microlitres d'une solution à 20 mg/ml d'acide (pyridyl 2 disulfanyl)-3 propionique dans le tertiobutanol, on ajoute 20 microlitres d'une solution à 60,3 mg/ml d'éthyl-1 (diméthylamino-3 propyl)-3 carbodiimide et on laisse 3 min à température ambiante. On ajoute 68 microlitres de la solution ainsi obtenue à 2 ml d'une solution d'anticorps à 8,9 mg/ml dans le tampon PBS. Le mélange est agité 15 min à 30 °C puis dialysé contre du tampon PBS à 4 °C. Après dialyse la solution protéique est centrifugée et l'on obtient ainsi 15 mg d'anticorps activé à une concentration de 7,9 mg/ml. Par dosage spectrophotométrique à 343 nm de la pyridine thione-2 libérée par échange avec le mercapto-2 éthanol, on constate que l'on a obtenu un anticorps portant 3,8 groupements disulfure mixte activé par mole d'anticorps.

III) Préparation de l'immunotoxine à chaîne de ricine à longue durée d'action.

A 1,5 ml de la solution d'anticorps activé précédemment obtenue (concentration 7,9 mg/ml, soit 11,8 mg d'anticorps activés), on ajoute 2 46 ml de chaîne A modifiée à 2,87 mg/ml et on incube 20 h à 20 °C. La solution est centrifugée puis purifiée par filtration sur colonne de Séphadex G100 avec mesure de la densité optique de l'effluent à 280 nm. Le regroupement des fractions contenant à la fois l'anticorps et la chaîne A conduit à 15 ml de solution d'immunotoxine à 0,7 mg/ml soit 10,5 mg. Cette solution contient 0,14 mg de chaîne A oxydée couplée à l'anticorps par ml.

Le taux de couplage moyen de cette préparation est donc de 1,2 mole de chaîne A oxydée par mole d'anticorps.

L'immunotoxine à chaîne A de ricine oxydée, IT (A-la) T101 obtenue comme indiqué ci-dessus a été étudiée en ce qui concerne ses propriétés pharmacocinétiques et ses propriétés de cytotoxicité spécifique vis-à-vis des cellules-cibles.

Exemple 5

Cet exemple démontre l'acquisition de la propriété de lente élimination plasmatique des immunotoxines à chaîne A de ricine à longue durée d'action désignées en abrégé IT (A-la).

I-Mode opératoire

Le conjugué préparé selon la procédure développée dans l'exemple 3 est administré chez le lapin par injection unique dans une veine de l'oreille. La quantité injectée correspond à 0,415 mg/kg exprimé en chaîne A. Les échantillons de sang sont prélevés au cours du temps sur héparine. Les plasmas sont analysés à l'aide d'un test immunoradiométrique à deux sites ci-après désigné par l'abréviation IRM-3.
Cet essai est réalisé selon la même technique que pour le test IRM-1 excepté que la solution Ac2 est ici une solution d'anticorps de chèvres anti-IgG de souris purifiés par chromatographie d'affinité et radiomarqués comme décrit pour la technique IRM-1. La mesure de la concentration en immunotoxine modifiée dans les échantillons à doser se fait par référence à une courbe d'étalonnage réalisée avec l'immunotoxine modifiée introduite à différentes concentrations connues. L'essai IRM-3 répond aux mêmes caractéristiques de fiabilité et de reproductibilité que celles décrites pour la technique IRM-1.
Pour comparaison une étude contrôle est réalisée dans les mêmes conditions avec le conjugué appelé IT-T101 obtenu par réaction entre le même anticorps T101 substitué par des groupements disulfure activé et la chaîne A native de ricine. La préparation et les propriétés cytotoxiques de ce conjugué ont été décrites dans la demande de brevet français n° 81/21836. Les résultats de ces expériences sont représentés de la même façon que pour la chaîne A de ricine non couplée dans l'exemple 1.

II-Résultats

La figure 4 montre les courbes d'élimination plasmatique en fonction du temps de l'IT-T101 et de l'IT (A-la) T101 injectées par voie intraveineuse. 24 h après l'injection, la concentration d'immunotoxine active est 140 fois supérieure pour l'IT (A-la) T101 que pour l'IT-T101. Ce fait démontre que les nouvelles propriétés pharmacocinétiques de la chaîne A oxydée sont conservées après couplage à un anticorps.

Exemple 6

Cet exemple démontre le maintien des propriétés de cytotoxicité spécifique de l'IT (A-la) T101 vis-à-vis des cellulescibles.

La propriété biologique fondamentale de la chaîne A de ricine est d'inhiber la synthèse protéique cellulaire par altération de la sous-unité ribosomale 60S. La technique utilisée met en oeuvre un modèle cellulaire dans lequel on mesure l'effet des substances étudiées sur l'incorporation de 14C-leucine dans les cellules cancéreuses en culture.

Les cellules utilisées appartiennent à la lignée cellulaire CEM issue d'une leucémie T humaine qui porte l'antigène T65. Les cellules sont incubées en présence de la substance à étudier puis, en fin d'incubation, on procède à la mesure du taux d'incorporation de 14C-leucine par les cellules ainsi traitées.

Cette mesure est effectuée selon une technique adaptée de la technique décrite dans Journal of Biological Chemistry 1974, 249 (11), 3557-3562 utilisant le traceur 14C-leucine pour la détermination du taux de synthèse protéique. La détermination de la radio-activité incorporée est effectuée ici sur les cellules entières isolées par filtration.

A partir de ces déterminations, on peut tracer les courbes effets/doses présentant en abscisse la concentration molaire en chaîne A des substances étudiées et en ordonnée l'incorporation de 14C-leucine exprimée en pourcentage de l'incorporation des cellules témoins en l'absence de toute substance affectant la synthèse protéique.

On peut ainsi déterminer pour chaque substance étudiée la concentration qui inhibe 50 % de l'incorporation de 14C-leucine ou "concentration inhibitrice 50" ($CI_{50}$).

La figure 5 représente les courbes obtenues dans la même expérience avec l'IT-(A-la)-T101 et la chaîne A oxydée non couplée en présence de chlorure d'ammonium 10 mM dans le milieu d'incubation. On peut constater sur cette figure que l'IT-(A-la)-T101 a une très forte activité cytotoxique ($CI_{50}$ = 5,5 x $10^{-12}$M) environ 80 000 fois supérieure à celle de la chaîne A oxydée non couplée mesurée dans les mêmes conditions.

Exemple 7

Cet exemple démontre l'efficacité cytotoxique comparée de l'IT-(A-la)-T101 et de l'IT-T101 vis-à-vis des cellules-cibles CEM, mesurée dans un test clonogénique.

La vocation des immunotoxines est l'éradication des cellules-cibles jusqu'à la dernière. Cette performance ne peut être évaluée qu'avec une technique d'une grande sensibilité : les tests d'inhibition de formation de colonies offrent cette possibilité puisqu'une seule cellule survivante peut être visualisée parmi plusieurs millions de cellules mortes. Ceci est rendu possible par des conditions de culture optimales en milieu gélifié, appliquées à la lignée lymphoïde humaine CEM.

I- Technique de mesure de la cytotoxicité par inhibition de la formation de colonies.

Le milieu utilisé pour le clonage est le milieu RPMI 1640 auquel on ajoute 1 mM d'alpha-cétoglutarate de sodium, 1 mM de oxaloacétate de sodium, 5 % de sérum de veau foetal inactivé et 10 % de sérum de cheval inactivé. Une première solution d'agar à 0,3 % (Agarose type VII, laboratoires SIGMA) est préparée dans ce milieu, disposée en couche mince dans de petites boîtes de Pétri et solidifiée à + 4° C. Les cellules sont mélangées à une seconde solution d'agar à 0,275 % maintenue à 37° C, déposée ensuite sur la première couche et solidifiée. Ces concentrations d'agar ont été choisies après une étude préliminaire visant à optimiser à la fois l'efficacité de clonage, la dimension des colonies et la consistance du milieu. Après 15 jours en incubateur, les colonies sont dénombrées à l'aide d'un compteur automatique de colonies ("ARTEK", DYNATECH, U.S.A.). L'établissement d'une droite d'étalonnage où sont portés le nombre de cellules ensemencées en fonction du nombre de colonies formées est indispensable pour déterminer l'efficacité du clonage et ainsi le nombre exact de cellules survivantes au traitement par l'immunotoxine. Nous avons vérifié que l'efficacité du clonage reflétée par cette droite étalon n'est pratiquement pas influencée par la présence d'une proportion élevée de cellules mortes, situation naturellement rencontrée quand les cellules sont traitées par l'immunotoxine.

Le traitement à l'immunotoxine est réalisé par incubation des cellules en phase de croissance exponentielle et à la concentration de $10^6$ /ml avec l'immunotoxine IT-(A-la)-T101 ou IT-T 101 à différentes concentrations, sous un volume total de 1 ml de milieu RPMI-1640 contenant 10 % de sérum de veau foetal inactivé, et 10 mM de chlorure d'ammonium. L'incubation a lieu à 37° C, sous atmosphère contenant 5 % de gaz carbonique et avec agitation horizontale des tubes tests (2 500 tr/min avec agitateur "GIRATORY G-2", NEW-BRUNSWICK). Les cellules sont ensuite lavées et différentes dilutions sont réalisées avant mélange à la solution d'agar, de façon que la lecture du nombre de cellules survivantes puisse être faite dans la zone de sensibilité maximale donnée par la droite étalon. Les résultats sont exprimés par le nombre absolu de cellules survivantes extrapolé à partir de l'efficacité de clonage, en utilisant la relation suivante :

nombre absolu de cellules survivantes :

$$\frac{C \times d}{E}$$

où C est le nombre de clones par boîte de Pétri, d est le facteur de dilution de la préparation cellulaire examinée, et E est l'efficacité de clonage établie à partir de la pente de la droite d'étalonnage. Chaque point correspond à la moyenne de trois essais.

II- Résultats

La figure 6 montre les courbes d'activité cytotoxique sur les cellules CEM des immunotoxines IT-(A-la)-T101 et IT-T101 en présence de chlorure d'ammonium à 10 mM en fonction de la concentration en immunotoxine (exprimée en molarité de chaîne A). Il apparaît que les efficacités de ces deux produits sont du même ordre de grandeur. La cytoréduction obtenue est extrêmement importante dans les deux cas puisque pour des concentrations aussi faibles que $10^{-11}$ M le taux de cellules survivantes résiduelles est de l'ordre de 0,001 % de la valeur initiale. Cet effet est hautement spécifique puisque, à ces concentrations, il a été vérifié que la chaîne A non couplée ou une immunotoxine non spécifique sont sans effet sur ces cellules.

Cet exemple démontre que l'IT-(A-la)-T101 a des propriétés de cytotoxicité spécifique pratiquement identiques à celles de l'IT-T 101 conventionnelle.

Exemple 8

Toxicité de la chaîne A à longue durée d'action injectée chez la souris.

Il était important de vérifier quel était l'impact toxicologique global sur l'animal entier de la chaîne A oxydée (la toxicité des immunotoxines étant du même ordre de grandeur que celle de la chaîne A à doses molaires égales). Pour cela, nous avons déterminé la dose létale 50 % de la chaîne A oxydée, administrée par voie intraveineuse chez la souris Charles River France CD1 en comparaison avec celle de la chaîne A native.

Les valeurs trouvées sont indiquées dans le tableau I.

Tableau I

| | $DL_{50}$ (microgrammes/souris) |
|---|---|
| chaîne A native | 550 |
| chaîne A oxydée | 800 |

Ces résultats montrent que la toxicité de la chaîne A oxydée est inférieure à celle de la chaîne A native. Ceci signifie qu'en dépit d'un accroissement considérable du taux plasmatique de la chaîne A lorsque celle-ci a été modifiée par oxydation la toxicité du produit n'est non seulement pas accrue, mais qu'au contraire elle est sensiblement diminuée.

On peut donc utiliser en tant que médicament en thérapeutique humaine les immunotoxines à sous-unités cytotoxiques modifiées. Ces immunotoxines modifiées peuvent être utilisées pour le traitement des affections cancéreuses ou non dont les cellules-cibles seraient reconnues par l'anticorps utilisé pour la préparation de l'immunotoxine. Les modalités optimales d'administration ainsi que la durée du traitement devront être déterminées dans chaque cas en fonction du sujet et de la nature de l'affection à traiter.

Les nouveaux médicaments selon l'invention sont conditionnés pour être utilisés par voie injectable et de préférence par voie intraveineuse.

## Revendications

### Revendications pour les Etats contractants suivants : BE, CH, DE, FR, GB, IT, LI, NL, SE

**1.** Une immunotoxine à longue durée d'action obtenue par couplage par une structure covalente divalente contenant un groupe disulfure ou thioéther entre d'une part un anticorps ou fragment d'anticorps, utilisé à l'état naturel ou correctement modifié, et d'autre part une glycoprotéine inactivant les ribosomes dont les motifs glucidiques sont modifiés par oxydation par l'ion periodate, les fonctions aldéhyde résultant de cette oxydation n'étant pas impliquées dans le couplage ci-dessus.

**2.** Une immunotoxine à longue durée d'action constituée par un conjugué dans lequel un anticorps ou fragment d'anticorps est couplé, par une structure covalente contenant un groupe disulfure ou thioéther, à une glycoprotéine inactivant les ribosomes à longue durée d'action cette dernière étant obtenue par traitement d'une glycoprotéine inactivant les ribosomes, dont les groupes thiol sont éventuellement protégés, avec une solution aqueuse d'un periodate alcalin pendant une période de temps de 0,2 à 24 h à la température de 0 à 15 °C et à l'abri de la lumière et par déblocage éventuel des groupes thiol, les fonctions aldéhyde résultant de cette oxydation n'étant pas impliquées dans le couplage ci-dessus.

**3.** Une immunotoxine ayant la formule statistique suivante :

P'-W-GPIR-la'

dans laquelle P' représente le radical d'une protéine P qui est un anticorps ou un fragment d'anticorps et dans lequel d'autres groupes fonctionnels sont éventuellement bloqués, GPIR-la' représente le radical d'une protéine GPIR-la qui est une glycoprotéine inactivant les ribosomes et dont les motifs glucidiques sont modifiés par oxydation par l'ion periodate et W représente une structure covalente divalente contenant un groupe thioéther ou un groupe disulfure dont les atomes de soufre sont soit

ceux des cystéines de P et de GPIR-la, soit sont liés aux fonctions propres de P et/ou de GPIR-la par des structures d'espacement portant un groupe fonctionnel lié auxdites fonctions propres de P et/ou de GPIR-la, à l'exception des fonctions aldéhyde résultant de l'oxydation de la gylcoprotéine par l'ion periodate.

4. Une immunotoxine ayant la formule statistique suivante :

P'-W-GPIR-la'

dans laquelle P' représente le radical d'une protéine P qui est un anticorps ou un fragment d'anticorps et dans lequel d'autres groupes fonctionnels sont éventuellement bloqués, GPIR-la' représente le radical d'une protéine qui est une glycoprotéine inactivant les ribosomes GPIR-la obtenue par traitement d'une glycoprotéine inactivant les ribosomes, dont les groupes thiol sont éventuellement protégés, avec une solution aqueuse d'un periodate alcalin pendant une période de temps de 0,2 à 24 h à la température ambiante et à l'abri de la lumière et par déblocage éventuel des groupes thiol, et dans lequel d'autres groupes fonctionnels sont éventuellement bloqués et W représente une structure covalente divalente contenant un groupe thioéther ou un groupe disulfure dont les atomes de soufre sont soit ceux des cystéines de P et GPIR-la, soit sont liés aux fonctions propres de P et/ou de GPIR-la par des structures d'espacement portant un groupe fonctionnel lié auxdites fonctions propres de P et/ou de GPIR-la à l'exception des fonctions aldéhyde résultant de l'oxydation de la glycoprotéine par l'ion periodate.

5. Une immunotoxine ayant la formule statistique suivante :

P'-W-GPIR-la'

dans laquelle P' et GPIR-la sont tels que définis dans la revendication 3, et W' représente une structure bivalente choisie parmi

(a) un groupement de formule

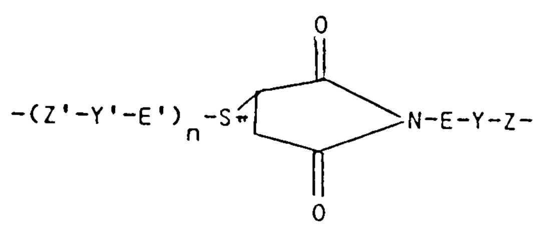

(b) un groupement de formule

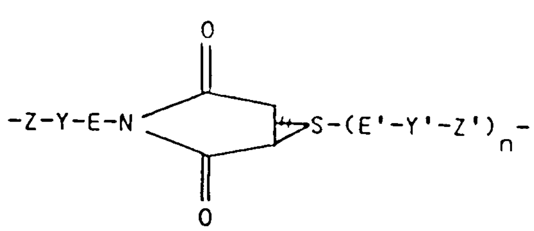

(c) un groupement de formule

-Z-Y-E-S-S-(E'-Y'-Z')$_n$-

(d) un groupement de formule

$-(Z'-Y'-E')_n-S-S-E-Y-Z-$

- Z et Z' représentent les fonctions propres des protéines GPIR-1a et P, choisies parmi l'atome d'oxygène provenant de l'hydroxyle de l'un des résidus de tyrosine ; le groupe carbonyle provenant de l'un des carboxyles terminaux ou de l'un des carboxyles libres des acides aspartiques et/ou glutamiques de GPIR-la et de P ; et le groupe -NH- provenant de l'une des amines terminales de GPIR-la et de P ou de l'une des amines en position epsilon de l'un des résidus de lysine ; et, seulement pour Z dans les structures covalentes (b) et (c), le groupe provenant de la structure dialdéhydique obtenue après oxydation d'une des structures glucidiques de P par l'acide periodique selon les méthodes connues ;
- Y et Y' représentent des groupes fonctionnels capables de se lier d'une façon covalente avec l'une quelconque des fonctions Z et Z' des protéines GPIR-la et P ;
- E et E' représentent des structures d'espacement inertes ;
- n représente zéro ou 1.

**6.** Une immunotoxine ayant la formule statistique suivante :

$P'(W'-A-la')_m$

dans laquelle m varie de 0,3 à 12, P' représente le radical d'une protéine P qui est un anticorps ou un fragment d'anticorps et dans lequel d'autres groupes fonctionnels sont éventuellement bloqués, A-la' représente le radical d'une glycoprotéine inactivant les ribosomes obtenue par traitement de la chaîne A de ricine, dont au moins un des groupes thiol de ses cystéines 171 et 257 est protégé, avec une solution aqueuse d'un periodate alcalin pendant une période de temps de 0,2 à 24 h à la température de 0 à 15°C et à l'abri de la lumière et par déprotection du groupe thiol ou des groupes thiol, dans ledit radical d'autres groupes fonctionnels étant éventuellement bloqués et W' représente une structure bivalente choisie parmi

(a) un groupement de formule

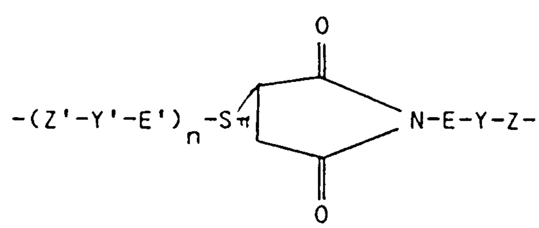

(b) un groupement de formule

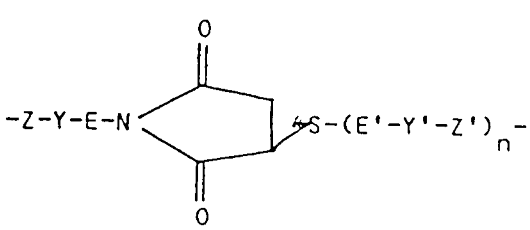

(c) un groupement de formule

$-Z-Y-E-S-S-(E'-Y'-Z')_n-$

(d) un groupement de formule

$-(Z'-Y'-E')_n-S-S-E-Y-Z-$

où

- Z, Z', Y, Y', E, E' et n sont tels que définis dans la revendication 5.

**7.** Une immunotoxine selon la revendication 6, de formule statistique :

$P'(W'-A-la')_m$

dans laquelle W' et A-la' sont tels que définis dans la revendication 6, P' est un fragment d'anticorps Fab ou Fab' et m varie de 0,3 à 2.

**8.** Une immunotoxine selon la revendication 6, de formule statistique :

$P'(W'-A-la')_m$

dans laquelle W' et A-la' sont tels que définis dans la revendication 6, P' est un fragment d'anticorps f-(ab')2 et m varie de 0,5 à 4.

**9.** Une immunotoxine selon la revendication 6 de formule statistique :

$P'(W'-A-la')_m$

dans laquelle W' et A-la' sont tels que définis dans la revendication 6, P' est un anticorps du type IgG et m varie de 0,5 à 6.

**10.** Une immunotoxine selon la revendication 6 de formule statistique :

$P'(W'-A-la')_m$

dans laquelle W' et A-la' sont tels que définis dans la revendication 6, P' est un anticorps du type IgM et m varie de 1 à 12.

**11.** Procédé pour la préparation d'une immunotoxine selon la revendication 1, caractérisé en ce que l'on forme, directement ou par l'intermédiaire d'une structure d'espacement, une liaison disulfure ou thioéther entre un anticorps ou fraction d'anticorps et une glycoprotéine inactivant les ribosomes dont les motifs glucidiques sont modifiés par oxydation par l'ion periodate, les fonctions aldéhyde résultant de cette oxydation n'étant pas impliquées dans la réaction de couplage ci-dessus.

**12.** Procédé pour la préparation d'une immunotoxine à longue durée d'action ayant une liaison covalente du type disulfure ou thioéther entre un anticorps et une glycoprotéine inactivant les ribosomes, caractérisé en ce que l'on forme une liaison disulfure ou thioéther entre un anticorps et une glycoprotéine inactivant les ribosomes à longue durée d'action obtenue par traitement d'une glycoprotéine inactivant les ribosomes, dont les groupes thiol sont éventuellement protégés, avec une solution aqueuse d'un periodate alcalin pendant une période de temps de 0,2 à 24 h à une température de 0 à 15 °C et à l'abri de la lumière et par déblocage éventuel des groupes thiol, les fonctions aldéhyde résultant de cette oxydation n'étant pas impliquées dans la réaction de couplage ci-dessus.

**13.** Procédé pour la préparation d'une immunotoxine selon la revendication 4, caractérisé en ce qu'on fait réagir en solution aqueuse et à la température ambiante une protéine $P_1$, qui est indifféremment soit la glycoprotéine inactivant les ribosomes à longue durée d'action GPIR-la, soit un anticorps ou fragment d'anticorps P, porteuse d'au moins un groupe thiol libre attaché à ladite protéine $P_1$ directement ou par l'intermédiaire d'une structure d'espacement, avec une protéine $P_2$, différente de $P_1$, qui est différemment soit la glycoprotéine inactivant les ribosomes à longue durée d'action GPIR-la, soit un anticorps ou fragment d'anticorps, porteuse d'un groupement capable de couplage avec le thiol libre de la protéine $P_1$, de façon à former une liaison thioéther ou disulfure.

**14.** Procédé pour la préparation d'une immunotoxine selon la revendication 5, caractérisé en ce qu'on fait réagir en solution aqueuse et à la température ambiante une protéine de formule :

$P_{1'}$-(Z-Y-E)$_n$SH IV

avec une protéine de formule :

$P_{2'}$-Z'-Y'-E'-G V

où $P_{1'}$ et $P_{2'}$ représentent les radicaux des protéines $P_1$ et $P_2$ liés aux fonctions propres desdites protéines ou, seulement lorsque $P_1$ et $P_2$ sont un anticorps ou fragment d'anticorps, les radicaux des protéines $P_1$ et $P_2$ provenant de l'ouverture des noyaux glucidiques par action de l'acide periodique, Z, Z', Y, Y', E et E' sont tels que définis ci-dessus et G représente un groupe

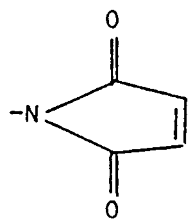

ou un groupe -S-S-X, où X est un groupe activateur.

**15.** Un produit de formule statistique :

GPIR-la''-O-CO-E-G IX

dans laquelle
- GPIR-la'' représente le radical d'une GPIR-la ou toute molécule dérivant de ladite GPIR-la par modification artificielle de l'un quelconque de ses groupements fonctionnels, privée d'un ou plusieurs groupes hydroxyles phénoliques des tyrosines ;
- l'atome d'oxygène est celui des groupes hydroxyles phénoliques manquant dans le radical $P_{2''}$;
- E représente une structure d'espacement inerte ; et
- G représente un groupe

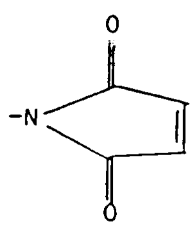

ou un groupe -S-S-X où X est un groupe activateur.

**Revendications pour l'Etat Contractant : AT**

**1.** Procédé pour l'obtention d'immunotoxines à longue durée d'action comportant un constituant glycopeptidique inactivant les ribosomes modifié sur ses motifs polysaccharidiques, caractérisé en ce qu'il consiste à former, directement ou par l'intermédiaire d'une structure d'espacement, une liaison disulfure ou thioéther entre un anticorps ou fragment d'anticorps, utilisé à l'état naturel ou correctement modifié, et une glycoprotéine inactivant les ribosomes dont les motifs glucidiques sont modifiés par oxydation par l'ion periodate, les fonctions aldéhyde résultant de cette oxydation n'étant pas impliquées dans la réaction de couplage ci-dessus.

2. Procédé selon la revendication 1, caractérisé en ce que la glycoprotéine inactivant les ribosomes à longue durée d'action est obtenue par traitement d'une glycoprotéine inactivant les ribosomes, dont les groupes thiol sont éventuellement protégés, avec une solution aqueuse d'un periodate alcalin pendant une période de temps de 0,2 à 24 h à la température de 0 à 15 ° C et à l'abri de la lumière et par déblocage éventuel des groupes thiol, les fonctions aldéhyde résultant de cette oxydation n'étant pas impliquées dans le couplage ci-dessus.

3. Procédé selon l'une des revendications 1 ou 2, pour l'obtention d'une immunotoxine ayant la formule statistique suivante :

P'-W-GPIR-la'

dans laquelle P' représente le radical d'une protéine P qui est un anticorps ou fragment d'anticorps et dans lequel d'autres groupes fonctionnels sont éventuellement bloqués, GPIR-la' représente le radical d'une protéine GPIR-la qui est une glycoprotéine inactivant les ribosomes et dont les motifs glucidiques sont modifiés par oxydation par l'ion periodate et W représente une structure covalente divalente contenant un groupe thioéther ou un groupe disulfure dont les atomes de soufre sont soit ceux des cystéines de P et de GPIR-la soit sont liés aux fonctions propres de P et/ou de GPIR-la par des structures d'espacement portant un groupe fonctionnel lié auxdites fonctions propres de P et/ou de GPIR-la, caractérisé en ce qu'il consiste à faire réagir en solution aqueuse et à la température ambiante une protéine $P_1$, qui est indifféremment soit la glycoprotéine inactivant les ribosomes à longue durée d'action GPIR-la, soit un anticorps ou fragment d'anticorps P, porteuse d'au moins un groupe thiol libre attaché à ladite protéine $P_1$, directement ou par l'intermédiaire d'une structure d'espacement, avec une protéine $P_2$, différente de $P_1$, qui est indifféremment soit la glycoprotéine inactivant les ribosomes à longue durée d'action GPIR-la, soit un anticorps ou fragment d'anticorps, porteuse d'un groupement capable de couplage avec le thiol libre de la protéine $P_1$, de façon à former une liaison thioéther ou disulfure.

4. Procédé selon l'une des revendications 1 ou 2, pour l'obtention d'une immunotoxine ayant la formule statistique suivante :

P'-W-GPIR-la'

dans laquelle P' représente le radical d'une protéine P qui est un anticorps ou un fragment d'anticorps et dans lequel d'autres groupes fonctionnels sont éventuellement bloqués, GPIR-la' représente le radical d'une protéine qui est une glycoprotéine inactivant les ribosomes GPIR-la, obtenue par traitement d'une glycoprotéine inactivant les ribosomes, dont les groupes thiol sont éventuellement protégés, avec une solution aqueuse d'un periodate alcalin pendant une période de temps de 0,2 à 24 h à la température ambiante et à l'abri de la lumière et par déblocage éventuel des groupes thiol, et dans lequel d'autres groupes fonctionnels sont éventuellement bloqués et W représente une structure covalente divalente contenant un groupe thioéther ou un groupe disulfure dont les atomes de soufre sont soit ceux des cystéines de P et GPIR-la, soit sont liés aux fonctions propres de P et/ou de GPIR-la par des structures d'espacement portant un groupe fonctionnel lié auxdites fonctions propres de P et/ou de GPIR-la, caractérisé en ce qu'il consiste à faire réagir en solution aqueuse et à la température ambiante une protéine $P_1$, qui est indifféremment soit la glycoprotéine inactivant les ribosomes à longue durée d'action GPIR-la, soit un anticorps ou fragment d'anticorps P, porteuse d'au moins un groupe thiol libre attaché à ladite protéine $P_1$ directement ou par l'intermédiaire d'une structure d'espacement, avec une protéine $P_2$, différente de $P_1$, qui est indifféremment soit la glycoprotéine inactivant les ribosomes à longue durée d'action GPIR-la, soit un anticorps ou fragment d' anticorps, porteuse d'un groupement capable de couplage avec le thiol libre de la protéine $P_1$, de façon à former une liaison thioéther ou disulfure.

5. Procédé selon l'une des revendications 1 ou 2, pour l'obtention d'une immunotoxine ayant la formule statistique suivante :

P'-W-GPIR-la'

dans laquelle P' et GPIR-la' sont tels que définis dans la revendication 3, et W représente une structure bivalente choisie parmi

(a) un groupement de formule

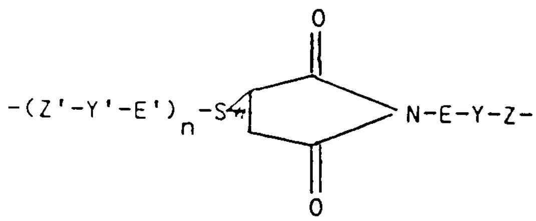

(b) un groupement de formule

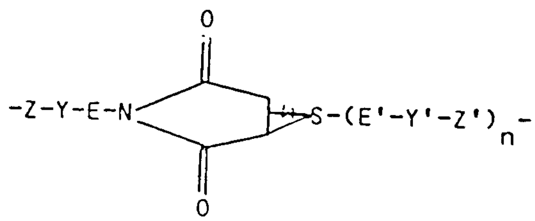

(c) un groupement de formule

-Z-Y-E-S-S-(E'-Y'-Z')$_n$-

(d) un groupement de formule

-(Z'-Y'-E')$_n$-S-S-E-Y-Z-

où

- Z et Z' représentent les fonctions propres des protéines GPIR-1a et P, choisies parmi l'atome d'oxygène provenant de l'hydroxyle de l'un des résidus de tyrosine ; le groupe carbonyle provenant de l'un des carboxyles terminaux ou de l'un des carboxyles libres des acides aspartiques et/ou glutamiques de GPIR-la et de P ; et le groupe -NH- provenant de l'une des amines terminales de GPIR-la et de P ou de l'une des amines en position epsilon de l'un des résidus de lysine ; et, seulement pour Z dans les structures covalentes (b) et (c), le groupe provenant de la structure dialdéhydique obtenue après oxydation d'une des structures glucidiques de P par l'acide périodique selon les méthodes connues ;
- Y et Y' représentent des groupes fonctionnels capables de se lier d'une façon covalente avec l'une quelconque des fonctions Z et Z' des protéines GPIR-la et P ;
- E et E' représentent des structures d'espacement inertes ;
- n représente zéro ou 1,

caractérisé en ce qu'on fait réagir en solution aqueuse et à la température ambiante une protéine de formule :

$P_{1'}$-(Z-Y-E)$_n$SH IV

avec une protéine de formule :

$P_{2'}$-Z'-Y'-E'-G V

où $P_{1'}$ et $P_{2'}$ représentent les radicaux des protéines $P_1$ et $P_2$ liés aux fonctions propres desdites protéines ou, seulement lorsque $P_1$ et $P_2$ sont un anticorps ou fragment d'anticorps, les radicaux des

protéines $P_1$ et $P_2$ provenant de l'ouverture des noyaux glucidiques par action de l'acide periodique, Z, Z', Y, Y', E et E' sont tels que définis ci-dessus et G représente un groupe

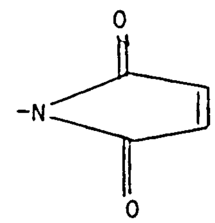

ou un groupe -S-S-X, où X est un groupe activateur.

**6.** Procédé selon l'une des revendications 1 ou 2, pour l'obtention d'une immunotoxine ayant la formule statistique suivante :

$P'(W'\text{-}A\text{-}la')_m$

dans laquelle m varie de 0,3 à 12, P' représente le radical d'une protéine P qui est un anticorps ou un fragment d'anticorps et dans lequel d'autres groupes fonctionnels sont éventuellement bloqués, A-la' représente le radical d'une glycoprotéine inactivant les ribosomes obtenue par traitement de la chaîne A de ricine, dont au moins un des groupes thiol de ses cystéines 171 et 257 est protégé, avec une solution aqueuse d'un periodate alcalin pendant une période de temps de 0,2 à 24 h à la température de 0 à 15 °C et à l'abri de la lumière et par déprotection du groupe thiol ou des groupes thiol, dans ledit radical d'autres groupes fonctionnels étant éventuellement bloqués et W' représente une structure bivalente choisie parmi

(a) un groupement de formule

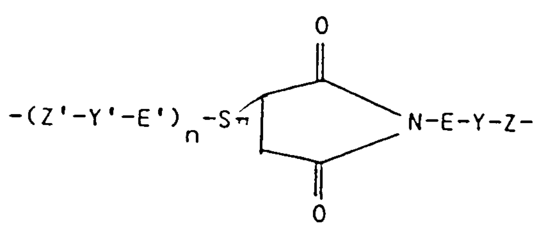

(b) un groupement de formule

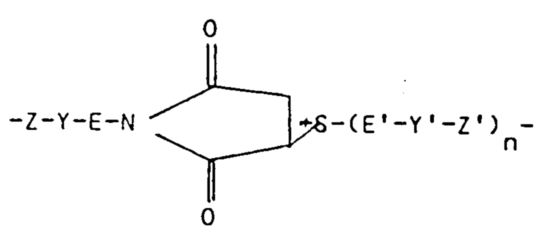

(c) un groupement de formule

$-Z\text{-}Y\text{-}E\text{-}S\text{-}S\text{-}(E'\text{-}Y'\text{-}Z')_n\text{-}$

(d) un groupement de formule

$-(Z'\text{-}Y'\text{-}E')_n\text{-}S\text{-}S\text{-}E\text{-}Y\text{-}Z\text{-}$

où Z, Z', Y, Y', E, E' et n sont tels que définis dans la revendication 5, caractérisé en ce qu'on fait réagir en solution aqueuse et à la température ambiante une protéine de formule :

$P_{1'}$-(Z-Y-E)$_n$SH IV

avec une protéine de formule :

$P_{2'}$-Z'-Y'-E'-G V

où $P_{1'}$ et $P_{2'}$ représentent les radicaux des protéines $P_1$ et $P_2$ liés aux fonctions propres desdites protéines ou, seulement lorsque $P_1$ et $P_2$ sont un anticorps ou fragment d'anticorps, les radicaux des protéines $P_1$ et $P_2$ provenant de l'ouverture des noyaux glucidiques par action de l'acide periodique, Z, Z', Y, Y', E et E' sont tels que définis ci-dessus et G représente un groupe

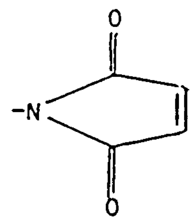

où un groupe -S-S-X, où X est un groupe activateur.

**7.** Procédé selon la revendication 6, caractérisé en ce que P' est un fragment d'anticorps Fab ou Fab' et m varie de 0,3 à 2.

**8.** Procédé selon la revendication 6, caractérisé en ce que P' est un fragment d'anticorps F(ab')$_2$ et m varie de 0,5 à 4.

**9.** Procédé selon la revendication 6, caractérisé en ce que P' est un anticorps du type IgG et m varie de 0,5 à 6.

**10.** Procédé selon la revendication 6, caractérisé en ce que P' est un anticorps du type IgM et m varie de 1 à 12.

**11.** Procédé pour l'obtention des produits intermédiaires ayant la formule statistique :

GPIR-la''-O-CO-E-G IX

dans laquelle

- GPIR-la'' représente le radical d'une GPIR-la ou toute molécule dérivant de ladite GPIR-la par modification artificielle de l'un quelconque de ses groupements fonctionnels, privée d'un ou plusieurs groupes hydroxyles phénoliques des tyrosines ;
- l'atome d'oxygène est celui des groupes hydroxyles phénoliques manquant dans le radical $P_{2''}$ ;
- E représente une structure d'espacement inerte ; et

- G représente un groupe

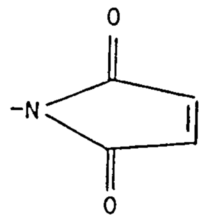

ou un groupe -S-S-X où X est un groupe activateur, caractérisé en ce qu'il consiste à faire réagir un produit de formule ;

GPIR-la''-OH

dans laquelle GPIR-la'' est tel que défini ci-dessus et le groupe hydroxyle est l'hydroxyle phénolique manquant dans les tyrosines du radical GPIR-la'', avec un composé de formule

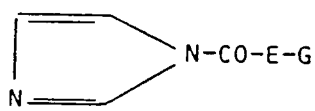

VI

dans laquelle E et G sont tels que définis ci-dessus à une température de 10 à 40 ° C dans un solvant aqueux contenant éventuellement un solvant organique miscible à l'eau.

## Claims

### Claims for the following Contracting States : BE, CH, DE, FR, GB, IT, LI, NL, SE, LU

1. A prolonged-action immunotoxin obtained by coupling by means of a divalent covalent structure containing a disulfide or thioether group between on the one hand, an antibody or antibody fragment used in the natural or correctly modified states, and on the other hand a glycoprotein which inactivates ribosomes, the carbohydrate units of which are modified by oxidation with the periodate ion, the aldehyde functions resulting from this oxidation not being involved in said coupling.

2. A prolonged-action immunotoxin constituted by a conjugate in which an antibody or antibody fragment is coupled, by means of a covalent structure containing a disulfide or thioether group, with a prolonged-action glycoprotein which inactivates ribosomes, said latter being obtained by treatment of a glycoprotein which inactivates ribosomes, the thiol groups of which are optionally protected, with an aqueous solution of an alkali metal periodate, for a period of 0.2 to 24 hours, at a temperature of 0 to 15 ° C and in the absence of light, and by unblocking of the thiol groups, if appropriate, the aldehyde functions resulting from this oxidation not being involved in said coupling.

3. An immunotoxin having the following statistical formula:

   P'-W-GPIR-la'

   in which P' represents the radical of a protein P, which is an antibody or an antibody fragment and in which other functional groups are optionally blocked, GPIR-la' represents the radical of a protein GPIR-la, which is a glycoprotein which inactivates ribosomes, the carbohydrate units of which are modified by oxidation with the periodate ion, and W represents a divalent covalent structure containing a thioether group or a disulfide group in which either the sulfur atoms are those of the cysteines of P and GPIR-la or they are bonded to the groups belonging to P and/or GPIR-la by spacing structures carrying a functional group bonded to the said groups belonging to P and/or GPIR-la, with the exception of the aldehyde functions resulting from the oxidation of glycoprotein with the periodate ion.

4. An immunotoxin having the following statistical formula:

P'-W-GPIR-la'

in which P' represents the radical of a protein P, which is an antibody or an antibody fragment and in which other functional groups are optionally blocked, GPIR-la' represents the radical of a protein which is a glycoprotein which inactivates ribosomes, GPIR-la, obtained by treatment of a glycoprotein which inactivates ribosomes, the thiol groups of which are optionally protected, with an aqueous solution of an alkali metal periodate, for a period of 0.2 to 24 hours, at room temperature and in the absence of light, and by unblocking of the thiol groups, if appropriate, and in which other functional groups are optionally blocked, and W represents a divalent covalent structure containing a thioether group or a disulfide group in which the sulfur atoms are either those of the cysteines of P and GPIR-la or they are bonded to the groups belonging to P and/or GPIR-la by spacing structures carrying a functional group bonded to the said groups belonging to P and/or GPIR-la, with the exception of the aldehyde functions resulting from the oxidation of glycoprotein with the periodate ion.

5. An immunotoxin having the following statistical formula:

P'-W'-GPIR-la'

in which P' and GPIR-la' are as defined in claim 3 and W' represents a divalent structure chosen from:
(a) a group of the formula:

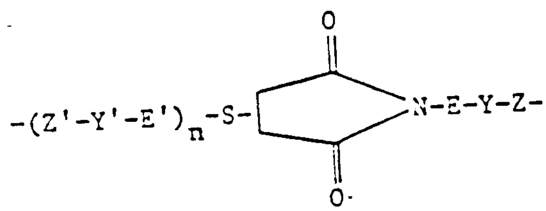

(b) a group of the formula:

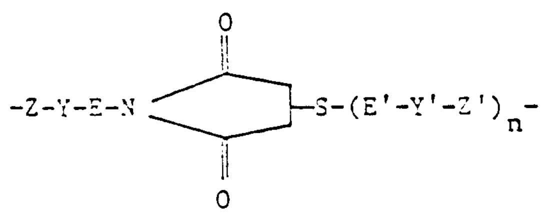

(c) a group of the formula:

$-Z-Y-E-S-S-(E'-Y'-Z')_n-$

(d) a group of the formula:

$-(Z'-Y'-E')_n-S-S-E-Y-Z-$,

in which:
- Z and Z' represent the groups belonging to the proteins GPIR-la and P, chosen from the oxygen atom originating from the hydroxyl of one of the tyrosine residues; the carbonyl group originating from one of the terminal carboxyls or one of the free carboxyls of the aspartic and/or glutamic acids of GPIR-la and P; and the -NH- group originating from one of the terminal amines of GPIR-la and P or from one of the amines in the epsilon position of one of the lysine residues; and, only

for Z in the covalent structures (b) and (c), the group originating from the dialdehyde structure obtained after oxidation of one of the carbohydrate structures of P with periodic acid according to the known methods;

- Y and Y' represent functional groups capable of bonding covalently with any one of the groups Z and Z' of the proteins GPIR-la and P;
- E and E' represent inert spacing structures;
- n represents zero or 1.

**6.** An immunotoxin having the following statistical formula:

P'(W'-A-la')$_m$

in which m varies from 0.3 to 12, P' represents the radical of a protein P, which is an antibody or an antibody fragment and in which other functional groups are optionally blocked, A-la' represents the radical of a glycoprotein which inactivates ribosomes, obtained by treatment of the A chain of ricin, in which at least one of the thiol groups of its cysteines 171 and 257 is protected, with an aqueous solution of an alkali metal periodate, for a period of 0.2 to 24 hours, at a temperature of 0 to 15 °C and in the absence of light, and by deprotection of the thiol group or thiol groups, other functional groups in the said radical being optionally blocked, and W' represents a divalent structure chosen from:

(a) a group of the formula:

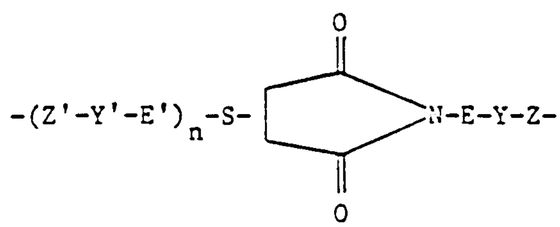

(b) a group of the formula:

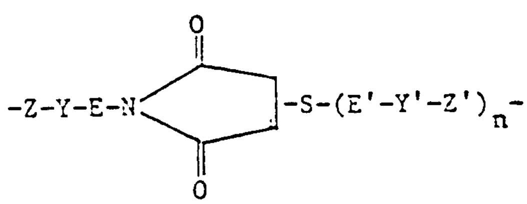

(c) a group of the formula:

-Z-Y-E-S-S-(E'-Y'-Z')$_n$-

(d) a group of the formula:

-(Z'-Y'-E')$_n$-S-S-E-Y-Z-

in which:

- Z, Z', Y, Y', E, E' and n are as defined in claim 5.

**7.** An immunotoxin according to claim 6, of the statistical formula:

P'(W'-A-la')$_m$

in which W' and A-la' are as defined in claim 6, P' is an antibody fragment Fab or Fab' and m varies from 0.3 to 2.

**8.** An immunotoxin according to claim 6, of the statistical formula:

$P'(W'\text{-}A\text{-}Ia')_m$

in which W' and A-la' are as defined in claim 6, P' is an antibody fragment $F(ab')_2$ and m varies from 0.5 to 4.

**9.** An immunotoxin according to claim 6, of the statistical formula:

$P'(W'\text{-}A\text{-}Ia')_m$

in which W' and A-la' are as defined in claim 6, P' is an antibody of the IgG type and m varies from 0.5 to 6.

**10.** An immunotoxin according to claim 6, of the statistical formula:

$P'(W'\text{-}A\text{-}Ia')_m$

in which W' and A-la' are as defined in claim 6, P' is an antibody of the IgM type and m varies from 1 to 12.

**11.** Process for the preparation of an immunotoxin according to claim 1, characterized in that a disulfide or thioether bond is formed, directly or via a spacing structure, between an antibody or antibody fraction and a glycoprotein which inactivates ribosomes, the carbohydrate units of which are modified by oxidation with the periodate ion, the aldehyde functions resulting from this oxidation not being involved in said coupling.

**12.** Process for the preparation of a prolonged-action immunotoxin having a covalent bond of the disulfide or thioether type between an antibody and a glycoprotein which inactives ribosomes, characterized in that a disulfide or thioether bond is formed between an antibody and a prolonged-action glycoprotein which inactivates ribosomes, obtained by treatment of a glycoprotein which inactivates ribosomes, the thiol groups of which are optionally protected, with an aqeuous solution of an alkali metal periodate, for a period of 0.2 to 24 hours, at a temperature of 0 to 15 °C and in the absence of light, and by unblocking of the thiol groups, if appropriate, the aldehyde functions resulting from this oxidation not being involved in said coupling.

**13.** Process for the preparation of an immunotoxin according to claim 4, characterized in that a protein $P_1$, which is arbitrarily either the prolonged-action glycoprotein which inactivates ribosomes, GPIR-la, or an antibody or antibody fragment P, carrying at least one free thiol group attached to the said protein $P_1$ directly or via a spacing structure, is reacted, in aqueous solution and at room temperature, with a protein $P_2$, which is different from $P_1$ and is arbitrarily either the prolonged-action glycoprotein which inactivates ribosomes, GPIR-la, or an antibody or antibody fragment, carrying a group capable of coupling with the free thiol of the protein $P_1$, so as to form a thioether or disulfide bond.

**14.** Process for the preparation of an immunotoxin according to claim 5, characterized in that a protein of the formula:

$P_{1'}\text{-}(Z\text{-}Y\text{-}E)_nSH$ IV

is reacted, in aqueous solution and at room temperature, with a protein of the formula:

$P_{2'}\text{-}Z'\text{-}Y'\text{-}E'\text{-}G$ V

in which $P_{1'}$ and $P_{2'}$ represent the radicals of the proteins $P_1$ and $P_2$ bonded to the groups belonging to the said proteins, or, only if $P_1$ and $P_2$ are an antibody or antibody fragment, the radicals of the proteins $P_1$ and $P_2$ originating from the opening of the carbohydrate nuclei by reaction with periodic

acid, Z, Z', Y, Y', E and E' are as defined above and G represents a group:

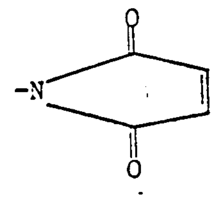

or a group -S-S-X, in which X is an activating group.

**15.** A product of the statistical formula:

GPIR-la"-O-CO-E-G IX

in which:
- GPIR-la" represents the radical of a GPIR-la or any molecule derived from the said GPIR-la by artificial modification of any one of its functional groups, from which one or more of the phenolic hydroxyl groups of the tyrosines have been removed;
- the oxygen atom is that belonging to the phenolic hydroxyl groups missing from the radical $P_{2''}$;
- E represents an inert spacing structure; and
- G represents a group:

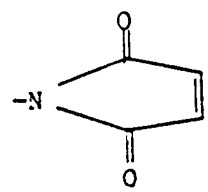

or a group -S-S-X, in which X is an activating group.

**Claims for the following Contracting State : AT**

**1.** Process for obtaining prolonged-action immunotoxins comprising a glycopeptidic constituent which inactivates ribosomes modified on its polysaccharide units, characterized in that it consists in forming, directly or by means of a spacing structure, a disulfide or thioether bond between an antibody or antibody fragment used in the natural or correctly modified state, and a glycoprotein which inactivates ribosomes, the carbohydrate units of which are modified by oxidation with the periodate ion, the aldehyde functions resulting from this oxidation not being involved in said coupling.

**2.** Process according to claim 1, characterized in that the prolonged-action glycoprotein which inactivates ribosomes is obtained by treatment of a glycoprotein which inactivates ribosomes, the thiol groups of which are optionally protected, with an aqueous solution of an alkali metal periodate, for a period of 0.2 to 24 hours, at a temperature of 0 to 15 ° C and in the absence of light, and by unblocking of the thiol groups, if appropriate, the aldehyde functions resulting from this oxidation not being involved in said coupling.

**3.** Process according to one of claims 1 or 2, for obtaining an immunotoxin having the following statistical formula:

P'-W-GPIR-la'

in which P' represents the radical of a protein P, which is an antibody or an antibody fragment and in which other functional groups are optionally blocked, GPIR-la' represents the radical of a protein GPIR-

Ia, which is a glycoprotein which inactivates ribosomes, the carbohydrate units of which are modified by oxidation with the periodate ion, and W represents a divalent covalent structure containing a thioether group or a disulfide group in which either the sulfur atoms are those of the cysteines of P and GPIR-Ia or they are bonded to the groups belonging to P and/or GPIR-Ia by spacing structures carrying a functional group bonded to the said groups belonging to P and/or GPIR-Ia, characterized in that it consists in reacting in aqueous solution and at room temperature, a protein $P_1$, which is, indifferently, either the prolonged-action glycoprotein which inactivates ribosomes, GPIR-Ia, or an antibody or antibody fragment P, carrying at least one free thiol group attached to said protein $P_1$, directly or by means of a spacing structure, with a protein $P_2$, different from $P_1$, which is indifferently either the prolonged-action glycoprotein which inactivates ribosomes, GPIR-Ia, or an antibody or antibody fragment, carrying a group capable of coupling with the free thiol of the protein $P_1$, so as to form a thioether or disulfide bond.

**4.** Process according to one of claims 1 or 2, for obtaining an immunotoxin having the following statistical formula:

P'-W-GPIR-Ia'

in which P' represents the radical of a protein P, which is an antibody or an antibody fragment and in which other functional groups are optionally blocked, GPIR-Ia' represents the radical of a protein which is a glycoprotein which inactivates ribosomes, GPIR-Ia, obtained by treatment of a glycoprotein which inactivates ribosomes, the thiol groups of which are optionally protected, with an aqueous solution of an alkali metal periodate, for a period of 0.2 to 24 hours, at room temperature and in the absence of light, and by unblocking of the thiol groups, if appropriate, and in which other functional groups are optionally blocked, and W represents a divalent covalent structure containing a thioether group or a disulfide group in which the sulfur atoms are either those of the cysteines of P and GPIR-Ia or they are bonded to the groups belonging to P and/or GPIR-Ia by spacing structures carrying a functional group bonded to the said groups belonging to P and/or GPIR-Ia, characterized in that it consists in reacting in aqueous solution and at room temperature, a protein $P_1$, which is, indifferently, either the prolonged-action glycoprotein which inactivates ribosomes, GPIR-Ia, or an antibody or antibody fragment P, carrying at least one free thiol group attached to said protein $P_1$, directly or by means of a spacing structure, with a protein $P_2$, different from $P_1$, which is indifferently either the prolonged-action glycoprotein which inactivates ribosomes, GPIR-Ia, or an antibody or antibody fragment, carrying a group capable of coupling with the free thiol of the protein $P_1$, so as to form a thioether or disulfide bond.

**5.** Process according to one of claims 1 or 2, for obtaning an immunotoxin having the following statistical formula:

P'-W'-GPIR-Ia'

in which P' and GPIR-Ia' are as defined in claim 3 and W' represents a divalent structure chosen from:
(a) a group of the formula:

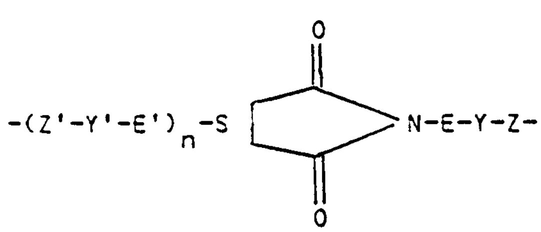

(b) a group of the formula:

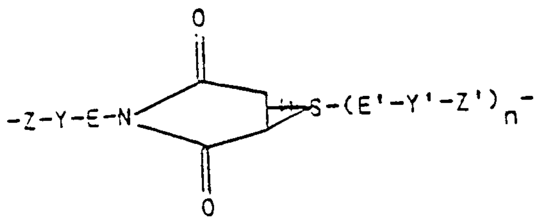

(c) a group of the formula:

$-Z-Y-E-S-S-(E'-Y'-Z')_n-$

(d) a group of the formula:

$-(Z'-Y'-E')_n-S-S-E-Y-Z-$,

in which:

- Z and Z' represent the groups belonging to the proteins GPIR-Ia and P, chosen from the oxygen atom originating from the hydroxyl of one of the tyrosine residues; the carbonyl group originating from one of the terminal carboxyls or one of the free carboxyls of the aspartic and/or glutamic acids of GPIR-Ia and P; and the -NH- group originating from one of the terminal amines of GPIR-Ia and P or from one of the amines in the epsilon position of one of the lysine residues; and, only for Z in the covalent structures (b) and (c), the group originating from the dialdehyde structure obtained after oxidation of one of the carbohydrate structures of P with periodic acid according to the known methods;
- Y and Y' represent functional groups capable of bonding covalently with any one of the groups Z and Z' of the proteins GPIR-Ia and P;
- E and E' represent inert spacing structures;
- n represents zero or 1,

characterized in that a protein of the formula:

$P_{1'}-(Z-Y-E)_nSH$ IV

is reacted, in aqueous solution and at room temperature, with a protein of the formula:

$P_{2'}-Z'-Y'-E'-G$ V

in which $P_{1'}$ and $P_{2'}$ represent the radicals of the proteins $P_1$ and $P_2$ bonded to the groups belonging to said proteins, or , only if $P_1$ and $P_2$ are an antibody or antibody fragment, the radicals of the proteins $P_1$ and $P_2$ originating from the opening of the carbohydrate nuclei by reaction with periodic acid, Z, Z', Y, Y', E and E' are as defined above and G represents a group:

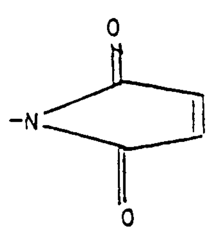

or a group -S-S-X, in which X is an activating group.

**6.** Process according to one of claims 1 or 2, for obtaining an immunotoxin having the following statistical formula:

$P'(W'-A-la')_m$

in which m varies from 0.3 to 12, P' represents the radical of a protein P, which is an antibody or an antibody fragment and in which other functional groups are optionally blocked, A-la' represents the radical of a glycoprotein which inactivates ribosomes, obtained by treatment of the A chain of ricin, in which at least one of the thiol groups of its cysteines 171 and 257 is protected, with an aqueous solution of an alkali metal periodate, for a period of 0.2 to 24 hours, at a temperature of 0 to 15 ° C and in the absence of light, and by deprotection of the thiol group or thiol groups, other functional groups in the said radical being optionally blocked, and W' represents a divalent structure chosen from:

(a) a group of the formula:

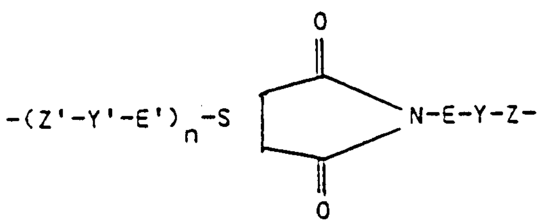

(b) a group of the formula:

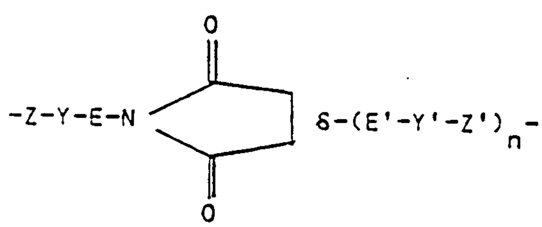

(c) a group of the formula:

$-Z-Y-E-S-S-(E'-Y'-Z')_n-$

(d) a group of the formula:

$-(Z'-Y'-E')_n-S-S-E-Y-Z-$

in which:

- Z, Z', Y, Y', E, E' and n are as defined in claim 5, characterized in that a protein of the formula:

$P_{1'}-(Z-Y-E)_nSH$ IV

is reacted, in aqueous solution and at room temperature, with a protein of the formula:

$P_{2'}-Z'-Y'-E'-G$ V

in which $P_{1'}$ and $P_{2'}$ represent the radicals of the proteins $P_1$ and $P_2$ bonded to the groups belonging to the said proteins, or , only if $P_1$ and $P_2$ are an antibody or antibody fragment, the radicals of the proteins $P_1$ and $P_2$ originating from the opening of the carbohydrate nuclei by reaction with periodic

acid, Z, Z', Y, Y', E and E' are as defined above and G represents a group:

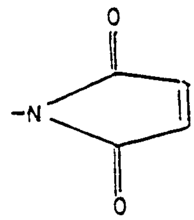

or a group -S-S-X, in which X is an activating group.

**7.** Process according to claim 6, characterized in that P' is an antibody fragment Fab or Fab' and m varies from 0.3 to 2.

**8.** Process according to claim 6, characterized in that P' is an antibody fragment $F(ab')_2$ and m varies from 0.5 to 4.

**9.** Process according to claim 6, characterized in that P' is an antibody of IgGtype and m varies from 0.5 to 6.

**10.** Process according to claim 6, characterized in that P' is an antibody of IgM type and m varies from 1 to 12.

**11.** Process for obtaining intermediate products of the statistical formula:

GPIR-la"-O-CO-E-G IX

in which:

- GPIR-la" represents the radical of a GPIR-la or any molecule derived from the said GPIR-la by artificial modification of any one of its functional groups, from which one or more of the phenolic hydroxyl groups of the tyrosines have been removed;
- the oxygen atom is that belonging to the phenolic hydroxyl groups missing from the radical $P_{2''}$;
- E represents an inert spacing structure; and
- G represents a group:

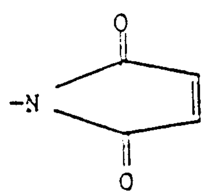

or a group -S-S-X, in which X is an activating group, characterized in that it consists in reacting a product of formula

GPIR-la"-OH

in which GPIR-la" is as defined above and the hydroxyl group is the phenol hydroxyle missing in the tyrosins of the radical GPIR-la", with a compound of formula

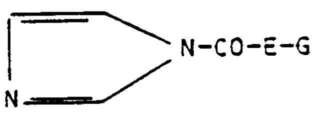

VI

in which E and G are as defined above at a temperature of 10 to 40 ° C in an aqueous solvent optionally containing a water-miscible organic solvent.

## Patentansprüche

### Patentansprüche für folgende Vertragsstaaten : BE, CH, DE, FR, GB, IT, LI, NL, SE, LU

1. Immunotoxin mit langer Wirkungsdauer, erhalten durch Kopplung eines im natürlichen Zustand verwendeten oder richtig modifizierten Antikörpers oder Antikörperfragments einerseits und eines Ribosomen inaktivierenden Glykoproteins anderseits, dessen Kohlenhydrateinheiten durch Oxidation mit einem Perjodation modifiziert sind, wobei die aus dieser Oxidation resultierenden Aldehydfunktionen bei der obigen Kopplung nicht zum Tragen kommen, mittels einer eine Disulfid- oder Thioethergruppe enthaltenden divalenten kovalenten Struktur.

2. Immunotoxin mit langer Wirkungsdauer, bestehend aus einem Konjugat, bei welchem ein Antikörper oder Antikörperfragment mittels einer eine Disulfid- oder Thioethergruppe enthaltenden kovalenten Struktur an ein Ribosomen inaktiviertendes Glykoprotein mit langer Wirkungdauer gekoppelt ist, wobei letzteres durch Behandlung eines Ribosomen inaktivierenden Glykoproteins, dessen Thiolgruppen gegebenenfalls geschützt sind, mit einer wässerigen Lösung eines Alkaliperjodats während eines Zeitraums von 0,2 bis 24 h bei einer Temperatur von 0 bis 15 ° C und vor Licht geschützt sowie gegebenenfalls durch Entblockung der Thiolgruppen erhalten ist, wobei die aus dieser Oxidation resultierenden Aldehydfunktionen bei der obigen Kopplung nicht zum Tragen kommen.

3. Immunotoxin mit der folgende Analysenformel:

   P'-W-GPIR-la'

   worin P' den Rest eines Proteins P darstellt, welcher ein Antikörper oder ein Antikörperfragment ist und bei dem andere funktionelle Gruppen gegebenenfalls blockiert sind, GPIR-la' den Rest eines Proteins GPIR-la darstellt, welcher ein Ribosomen inaktivierendes Protein ist und dessen Kohlenhydrateinheiten durch Oxidation mit einem Perjodation modifiziert sind, und W eine divalente kovalente Struktur darstellt, welche einen Thioether oder eine Disulfidgruppe enthält, deren Schwefelatome entweder jene der Cysteine von P und GPIR-la oder an die eigentlichen Funktionen von P und/oder GPIR-la mittels Raumstrukturen gebunden sind, die eine funktionelle Gruppe tragen, welche an die eigentlichen Funktionen von P und/oder GPIR-la gebunden ist, mit Ausnahme der aus der Oxidation des Glycoproteins mit dem Perjodation resultierenden Aldehydfunktionen.

4. Immunotoxin mit der folgenden Analysenformel

   P'-W-GPIR-la'

   worin P' den Rest eines Proteins P darstellt, welcher ein Antikörper oder ein Antikörperfragment ist und bei dem andere funktionelle Gruppen gegebenenfalls blockiert sind, GPIR-la' den Rest eines proteins GPIR-la darstellt, welcher ein Ribosomen inaktivierendes Glycoprotein ist, erhalten durch Behandlung eines Ribosomen inaktivierenden Glycoproteins, dessen Thiolgruppen gegebenenfalls geschützt sind, mit einer wässerigen Lösung eines Alkaliperjodats während eines Zeitraums von 0,2 bis 24 h bei Umgebungstemperatur und vor Licht geschützt sowie gegebenenfalls durch Entblockung der Thiolgruppen, und bei welchem andere funktionelle Gruppen gegebenenfalls blockiert sind, und W eine divalente kovalente Struktur darstellt, welche einen Thioether oder eine Disulfidgruppe enthält, deren Schwefelatome entweder jene der Cysteine von P und GPIR-la oder an die eigentlichen Funktionen von P

und/oder GPIR-la mittels Raumstrukturen gebunden sind, die eine funktionelle Gruppe tragen, welche an die eigentlichen Funktionen von P und/oder von GPIR-la gebunden ist, mit Ausnahme der aus der Oxidation des Glycoproteins mit dem Perjodation resultierenden Aldehydfunktionen.

**5.** Immunotoxin der folgenden Analysenformel:

P'-W-GPIR-la'

worin P' und GPIR-la' wie in Anspruch 3 definiert sind und W' eine bivalente Struktur darstellt, ausgewählt aus
(a) einer Gruppe der Formel

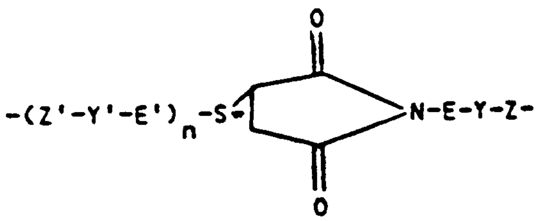

(b) einer Gruppe der Formel

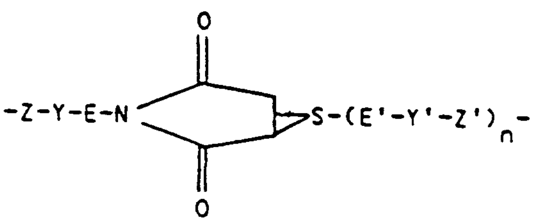

(c) einer Gruppe der Formel

-Z-Y-E-S-S-(E'-Y'-Z')$_n$-

(d) einer Gruppe der Formel

-(Z'-Y'-E')$_n$-S-S-E-Y-Z-

- Z und Z' die eigentlichen Funktionen der Proteine GPIR-la und P darstellen, ausgewählt aus dem vom Hydroxyl eines der Tyrosinreste kommenden Sauerstoffatom; der von einem der terminalen Carboxyle oder von einem der freien Carboxyle der Asparagin- und/oder Glutaminsäure von GPIR-la und P kommenden Carbonylgruppe; und der von einem der terminalen Amine von GPIR-la und von P oder einem der Amine in Position Epsilon eines der Lysinreste kommenden Gruppe -NH-; und nur für Z in den kovalenten Strukturen (b) und (c) der von der nach Oxidation einer der Kohlenhydratstrukturen von P mittels Perjodsäure nach bekannten Methoden erhaltenen Dialdehydstruktur kommenden Gruppe;
- Y und Y' funktionelle Gruppen darstellen, die sich auf kovalente Weise mit irgendeiner der Funktionen Z und Z' der Proteine GPIR-la und P binden können;
- E und E' inerte Raumstrukturen darstellen;
- n für Null oder 1 steht.

**6.** Immunotoxin mit der folgenden Analysenformel

P'(W'-A-la')$_m$

in welcher m von 0,3 bis 12 variiert, P' den Rest eines Proteins P darstellt, welcher ein Antikörper oder ein Antikörperfragment ist und bei dem andere funktionelle Gruppen gegebenenfalls blockiert sind, A-la' den Rest eines Ribosomen inaktivierenden Glycoproteins ist, erhalten durch Behandlung der Kette A von Ricin, bei der mindestens eine der Thiolgruppen ihrer Cysteine 171 und 257 geschützt ist, mit einer wässerigen Lösung eines Alkaliperjodats während eines Zeitraums von 0,2 bis 24 h bei einer Temperatur von 0 bis 15 °C und vor Licht geschützt sowie durch Entschützung der Thiolgruppe(n), wobei in diesem Rest andere funktionelle Gruppen gegebenenfalls blockiert sind, und W' eine bivalente Struktur darstellt, ausgewählt aus

(a) einer Gruppe der Formel

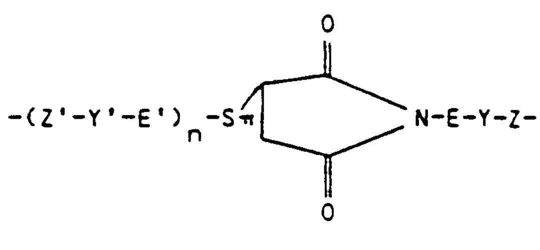

(b) einer Gruppe der Formel

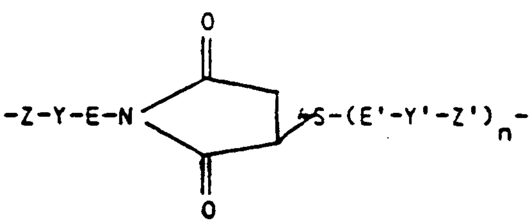

(c) einer Gruppe der Formel

-Z-Y-E-S-S-(E'-Y'-Z')$_n$-

(d) einer Gruppe der Formel

-(Z'-Y'-E')$_n$-S-S-E-Y-Z-

worin

- Z, Z', Y, Y', E, E' und n wie in Anspruch 5 definiert sind.

**7.** Immunotoxin nach Anspruch 6 der Analysenformel

P'(W'-A-la')$_m$

in welcher W' und A-la' wie in Anspruch 6 definiert sind, P' ein Antikörperfragment Fab oder Fab' ist und m von 0,3 bis 2 variiert.

**8.** Immunotoxin nach Anspruch 6 der Analysenformel

P'(W'-A-la')$_m$

in welcher W' und A-la' wie in Anspruch 6 definiert sind, P' ein Antikörperfragment F(ab')2 ist und m von 0,5 bis 4 variiert.

**9.** Immunotoxin nach Anspruch 6 der Analysenformel

P'(W'-A-la')$_m$

in welcher W' und A-la' wie in Anspruch 6 definiert sind, P' ein Antikörper der Type IgG ist und m von 0,5 bis 6 variiert.

**10.** Immunotoxin nach Anspruch 6 der Analysenformel

$P'(W'\text{-}A\text{-}la')_m$

in welcher W' und A-la' wie in Anspruch 6 definiert sind, P' ein Antikörper der Type IgM ist und m von 1 bis 12 variiert.

**11.** Verfahren zur Herstellung eines Immunotoxins nach Anspruch 1, dadurch gekennzeichnet, daß man direkt oder über eine Raumstruktur eine Disulfid- oder Etherbindung zwischen einem Antikörper oder Antikörperfragment und einem Ribosomen inaktivierenden Glycoprotein, dessen Kohlenhydrateinheiten durch Oxidation mit einem Perjodation modifiziert sind, wobei die aus dieser Oxidation resultierenden Aldehydfunktionen bei der obigen Kopplungsreaktion nicht zum Tragen kommen, bildet.

**12.** Verfahren zur Herstellung eines Immunotoxins mit langer Wirkungsdauer, umfassend eine kovalente Bindung der Disulfid- oder Thioethertype zwischen einem Antikörper und einem Ribosomen inaktivierenden Glycoprotein, dadurch gekennzeichnet, daß man eine Disulfid- oder Thioetherbindung zwischen einem Antikörper und einem Ribosomen inaktivierenden Glycoprotein mit langer Wirkungsdauer, erhalten durch Behandlung eines Ribosomen inaktivierenden Glykoproteins, dessen Thiolgruppen gegebenenfalls geschützt sind, mit einer wässerigen Lösung eines Alkaliperjodats während eines Zeitraums von 0,2 bis 24 h bei einer Temperatur von 0 bis 15 ° C und vor Licht geschützt sowie gegebenenfalls durch Entblockung der Thiolgruppen, wobei die aus dieser Oxidation resultierenden Aldehydfunktionen bei der obigen Kopplungsreaktion nicht zum Tragen kommen, bildet.

**13.** Verfahren zur Herstellung eines Immunotoxins nach Anspruch 4, dadurch gekennzeichnet, daß man in wässeriger Lösung und bei Umgebungstemperatur ein Protein $P_1$, welches ohne Unterschied entweder das Ribosomen inaktivierende Glycoprotein mit langer Wirkungsdauer GPIR-la oder ein Antikörper oder Antikörperfragment P und Träger mindestens einer direkt oder über eine Raumstruktur am Protein $P_1$ hängenden freien Thiolgruppe ist, mit einem Protein $P_2$, welches von $P_1$ unterschiedlich und ohne Unterschied entweder das Ribisomen inaktivierende Glycoprotein mit langer Wirkungsdauer GPIR-la oder ein Antikörper oder Antikörperfragment und Träger einer mit der freien Thiolgruppe des Proteins $P_1$ kopplungsfähigen Gruppe ist, reagieren läßt, sodaß eine Thioether- oder Disulfidbindung gebildet wird.

**14.** Verfahren zur Herstellung eines Immunotoxins nach Anspruch 5, dadurch gekennzeichnet, daß man in wässeriger Lösung und bei Umgebungstemperatur ein Protein der Formel:

$P_{1'}\text{-}(Z\text{-}Y\text{-}E)_nSH$ IV

mit einem Protein der Formel

$P_{2'}\text{-}Z'\text{-}Y'\text{-}E'\text{-}G$ V

worin $P_1$ und $P_2$ die Reste der Proteine $P_1$ und $P_2$, die an die eigentlichen Funktionen der Proteine gebunden sind, oder, nur wenn $P_1$ und $P_2$ ein Antikörper oder Antikörperfragment sind, die Reste der Proteine $P_1$ und $P_2$, die von der Öffnung der Kohlenhydratkerne durch Einwirken von Perjodatsäure

kommen, darstellen, Z, Z', Y, Y', E und E' wie oben definiert sind und G eine Gruppe

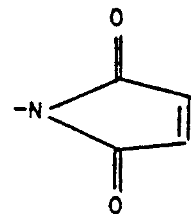

oder eine Gruppe -S-S-X, worin X eine Aktivatorgruppe ist, bedeutet.

**15.** Produkt der Analysenformel

GPIR-la"-O-CO-E-G IX

worin

- GPIR-la" den Rest eines GPIR-la oder jedes Moleküls, das von GPIR-la durch künstliche Modifikation irgendeiner seiner funktionellen Gruppen, dem eine oder mehrere phenolische Hydroxylgruppen der Tyrosine entzogen sind, darstellt;
- das Sauerstoffatom jenes der phenolischen Hydroxylgruppen ist, die im Rest $P_{2''}$ fehlen;
- E eine inerte Raumstruktur darstellt; und
- G für eine Gruppe

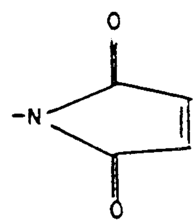

oder eine Gruppe -S-S-X, worin X eine Aktivatorgruppe ist, steht.

**Patentansprüche für folgenden Vertragsstaat : AT**

**1.** Verfahren zur Herstellung von Immunotoxinen mit langer Wirkungsdauer, umfassend einen an seinen Polysaccharideinheiten modifizierten, Ribosomen inaktivierenden Glycopeptidbestandteil, dadurch gekennzeichnet, daß es darin besteht, daß direkt oder über eine Raumstruktur eine Disulfid- oder Etherbindung zwischen einem im natürlichen Zustand oder richtig modifiziert verwendeten Antikörper oder Antikörperfragment und einem Ribosomen inaktivierenden Glycoprotein, dessen Kohlenhydrateinheiten durch Oxidation mit einem Perjodation modifiziert sind, wobei die aus dieser Oxidation resultierenden Aldehydfunktionen bei der obigen Kopplungsreaktion nicht zum Tragen kommen, gebildet wird.

**2.** Verfahren nach Anspruch 1, dadurch gekennzeichnet, daß das Ribosomen inaktivierende Glycoprotein mit langer Wirkungsdauer erhalten wird durch Behandlung eines Ribosomen inaktivierenden Glykoproteins, dessen Thiolgruppen gegebenenfalls geschützt sind, mit einer wässerigen Lösung eines Alkaliperjodats während eines Zeitraums von 0,2 bis 24 h bei einer Temperatur von 0 bis 15 °C und vor Licht geschützt sowie gegebenenfalls durch Entblockung der Thiolgruppen, wobei die aus dieser Oxidation resultierenden Aldehydfunktionen bei der obigen Kopplung nicht zum Tragen kommen.

**3.** Verfahren nach einem der Ansprüche 1 oder 2 zur Herstellung eines Immunotoxins mit der folgenden Analysenformel:

P'-W-GPIR-la'

worin P' den Rest eines Proteins P darstellt, welcher ein Antikörper oder ein Antikörperfragment ist und

bei dem andere funktionelle Gruppen gegebenenfalls blockiert sind, GPIR-la' den Rest eines Proteins GPIR-la darstellt, welcher ein Ribosomen inaktivierendes Protein ist und dessen Kohlenhydrateinheiten durch Oxidation mit einem Perjodation modifiziert sind, und W eine divalente kovalente Struktur darstellt, welche einen Thioether oder eine Disulfidgruppe enthält, deren Schwefelatome entweder jene der Cysteine von P und GPIR-la oder an die eigentlichen Funktionen von P und/oder GPIR-la mittels Raumstrukturen gebunden sind, die eine funktionelle Gruppe tragen, welche an die eigentlichen Funktionen von P und/oder GPIR-la gebunden ist, dadurch gekennzeichnet, daß es darin besteht, daß man in wässeriger Lösung und bei Umgebungstemperatur ein Protein $P_1$, welches ohne Unterschied entweder das Ribosomen inaktivierende Glycoprotein mit langer Wirkungsdauer GPIR-la oder ein Antikörper oder Antikörperfragment P und Träger mindestens einer direkt oder über eine Raumstruktur am Protein $P_1$ hängenden freien Thiolgruppe ist, mit einem Protein $P_2$, welches von $P_1$ unterschiedlich und ohne Unterschied entweder das Ribisomen inaktivierende Glycoprotein mit langer Wirkungsdauer GPIR-la oder ein Antikörper oder Antikörperfragment und Träger einer mit der freien Thiolgruppe des Proteins $P_1$ kopplungsfähigen Gruppe ist, reagieren läßt, sodaß eine Thioether- oder Disulfidbindung gebildet wird.

**4.** Verfahren nach einem der Ansprüche 1 oder 2 zur Herstellung eines Immunotoxins mit der folgenden Analysenformel

P'-W-GPIR-la'

worin P' den Rest eines Proteins P darstellt, welcher ein Antikörper oder ein Antikörperfragment ist und bei dem andere funktionelle Gruppen gegebenenfalls blockiert sind, GPIR-la' den Rest eines Proteins GPIR-la darstellt, welcher ein Ribosomen inaktivierendes Glycoprotein ist, erhalten durch Behandlung eines Ribosomen inaktivierenden Glycoproteins, dessen Thiolgruppen gegebenenfalls geschützt sind, mit einer wässerigen Lösung eines Alkaliperjodats während eines Zeitraums von 0,2 bis 24 h bei Umgebungstemperatur und vor Licht geschützt sowie gegebenenfalls durch Entblockung der Thiolgruppen, und bei welchem andere funktionelle Gruppen gegebenenfalls blockiert sind, und W eine divalente kovalente Struktur darstellt, welche einen Thioether oder eine Disulfidgruppe enthält, deren Schwefelatome entweder jene der Cysteine von P und GPIR-la oder an die eigentlichen Funktionen von P und/oder GPIR-la mittels Raumstrukturen gebunden sind, die eine funktionelle Gruppe tragen, welche an die eigentlichen Funktionen von P und/oder GPIR-la gebunden ist, dadurch gekennzeichnet, daß es darin besteht, daß man in wässeriger Lösung und bei Umgebungstemperatur ein Protein $P_1$, welches ohne Unterschied entweder das Ribosomen inaktivierende Glycoprotein mit langer Wirkungsdauer GPIR-la oder ein Antikörper oder Antikörperfragment P und Träger mindestens einer direkt oder über eine Raumstruktur am Protein $P_1$ hängenden freien Thiolgruppe ist, mit einem Protein $P_2$, welches von $P_1$ unterschiedlich und ohne Unterschied entweder das Ribisomen inaktivierende Glycoprotein mit langer Wirkungsdauer GPIR-la oder ein Antikörper oder Antikörperfragment und Träger einer mit der freien Thiolgruppe des Proteins $P_1$ kopplungsfähigen Gruppe ist, reagieren läßt, sodaß eine Thioether- oder Disulfidbindung gebildet wird.

**5.** Verfahren nach einem der Ansprüche 1 oder 2 zur Herstellung eines Immunotoxins der folgenden Analysenformel:

P'-W-GPIR-la'

worin P' und GPIR-la' wie in Anspruch 3 definiert sind und W eine bivalente Struktur darstellt, ausgewählt aus

(a) einer Gruppe der Formel

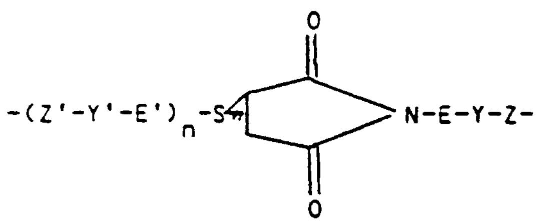

(b) einer Gruppe der Formel

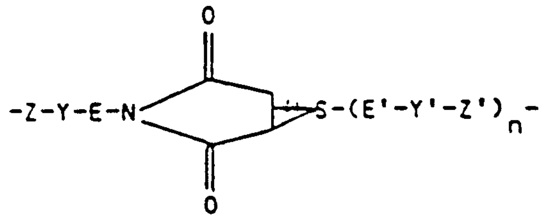

(c) einer Gruppe der Formel

-Z-Y-E-S-S-(E'-Y'-Z')$_n$-

(d) einer Gruppe der Formel

-(Z'-Y'-E')$_n$-S-S-E-Y-Z-

worin

- Z und Z' die eigentlichen Funktionen der Proteine GPIR-Ia und P darstellen, ausgewählt aus dem vom Hydroxyl eines der Tyrosinreste kommenden Sauerstoffatom; der von einem der terminalen Carboxyle oder von einem der freien Carboxyle der Asparagin- und/oder Glutaminsäure von GPIR-Ia und P kommenden Carbonylgruppe; und der von einem der terminalen Amine von GPIR-Ia und von P oder einem der Amine in Position Epsilon eines der Lysinreste kommenden Gruppe -NH-; und nur für Z in den kovalenten Strukturen (b) und (c) der von der nach Oxidation einer der Kohlenhydratstrukturen von P mittels Perjodsäure nach bekannten Methoden erhaltenen Dialdehydstruktur kommenden Gruppe;
- Y und Y' funktionelle Gruppen darstellen, die sich auf kovalente Weise mit irgendeiner der Funktionen Z und Z' der Proteine GPIR-Ia und P binden können;
- E und E' inerte Raumstrukturen darstellen;
- n für Null oder 1 steht, dadurch gekennzeichnet, daß man in wässeriger Lösung und bei Umgebungstemperatur ein Protein der Formel:

  $P_{1'}$-(Z-Y-E)$_n$SH IV

  mit einem Protein der Formel

  $P_{2'}$-Z'-Y'-E'-G V

worin $P_1$ und $P_2$ die Reste der Proteine $P_1$ und $P_2$, die an die eigentlichen Funktionen der Proteine gebunden sind, oder, nur wenn $P_1$ und $P_2$ ein Antikörper oder Antikörperfragment sind, die Reste der Proteine $P_1$ und $P_2$, die von der Öffnung der Kohlenhydratkerne durch Einwirken von Perjodatsäure kommen, darstellen, Z, Z', Y, Y', E und E' wie oben definiert sind und G eine Gruppe

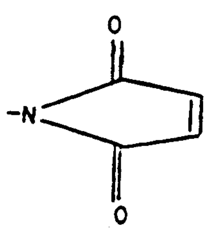

oder eine Gruppe -S-S-X, worin X eine Aktivatorgruppe ist, bedeutet.

6. Verfahren nach einem der Ansprüche 1 oder 2 zur Herstellung eines Immunotoxins mit der folgenden Analysenformel

P'(W'-A-la')$_m$

in welcher m von 0,3 bis 12 variiert, P' den Rest eines Proteins p darstellt, welcher ein Antikörper oder ein Antikörperfragment ist und bei dem andere funktionelle Gruppen gegebenenfalls blockiert sind, A-la' den Rest eines Ribosomen inaktivierenden Glycoproteins ist, erhalten durch Behandlung der Kette A von Ricin, bei der mindestens eine der Thiolgruppen ihrer Cysteine 171 und 257 geschützt ist, mit einer wässerigen Lösung eines Alkaliperjodats während eines Zeitraums von 0,2 bis 24 h bei einer Temperatur von 0 bis 15 °C und vor Licht geschützt sowie durch Entschützung der Thiolgruppe(n), wobei in diesem Rest andere funktionelle Gruppen gegebenenfalls blockiert sind, und W' eine bivalente Struktur darstellt, ausgewählt aus

(a) einer Gruppe der Formel

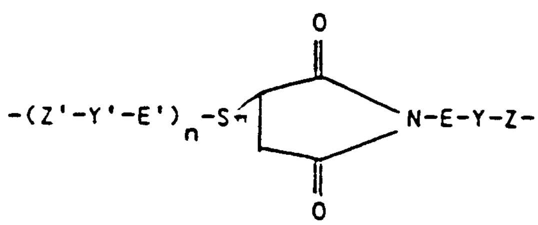

(b) einer Gruppe der Formel

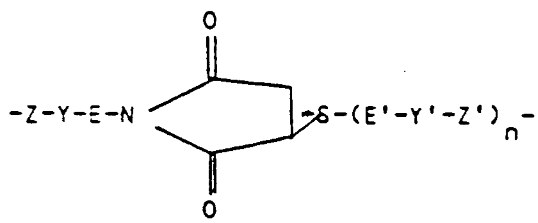

(c) einer Gruppe der Formel

-Z-Y-E-S-S-(E'-Y'-Z')$_n$-

(d) einer Gruppe der Formel

-(Z'-Y'-E')$_n$-S-S-E-Y-Z-

worin

- Z, Z', Y, Y', E, E' und n wie in Anspruch 5 definiert sind,

dadurch gekennzeichnet, daß man in wässeriger Lösung und bei Umgebungstemperatur ein Protein der Formel:

$P_{1'}$-(Z-Y-E)$_n$SH IV

mit einem Protein der Formel

$P_{2'}$-Z'-Y'-E'-G V

worin $P_1$ und $P_2$ die Reste der Proteine $P_1$ und $P_2$, die an die eigentlichen Funktionen der Proteine gebunden sind, oder, nur wenn $P_1$ und $P_2$ ein Antikörper oder Antikörperfragment sind, die Reste der Proteine $P_1$ und $P_2$, die von der Öffnung der Kohlenhydratkerne durch Einwirken von Perjodatsäure

kommen, darstellen, Z, Z', Y, Y', E und E' wie oben definiert sind und G eine Gruppe

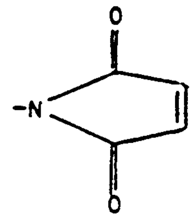

oder eine Gruppe -S-S-X, worin X eine Aktivatorgruppe ist, bedeutet.

**7.** Verfahren nach Anspruch 6, dadurch gekennzeichnet, daß P' ein Antikörperfragment Fab oder Fab' ist und m von 0,3 bis 2 variiert.

**8.** Verfahren nach Anspruch 6, dadurch gekennzeichnet, daß P' ein Antikörperfragment F(ab')2 ist und m von 0,5 bis 4 variiert.

**9.** Verfahren nach Anspruch 6, dadurch gekennzeichnet, daß P' ein Antikörper der Type IgG ist und m von 0,5 bis 6 variiert.

**10.** Verfahren nach Anspruch 6, dadurch gekennzeichnet, daß P' ein Antikörper der Type IgM ist und m von 1 bis 12 variiert.

**11.** Verfahren zur Herstellung von Zwischenprodukten mit der Analysenformel:

GPIR-la''-O-CO-E-G IX

worin
- GPIR-la'' den Rest eines GPIR-la oder jedes Moleküls, das von GPIR-la durch künstliche Modifikation irgendeiner seiner funktionellen Gruppen, dem eine oder mehrere phenolische Hydroxylgruppen der Tyrosine entzogen sind, darstellt;
- das Sauerstoffatom jenes der phenolischen Hydroxylgruppen ist, die im Rest $P_{2''}$ fehlen;
- E eine inerte Raumstruktur darstellt; und
- G für eine Gruppe

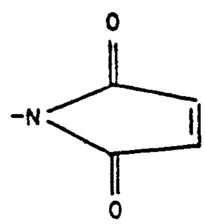

oder eine Gruppe -S-S-X, worin X eine Aktivatorgruppe ist, steht,

dadurch gekennzeichnet, daß es darin besteht, daß ein Produkt der Formel

GPIR-la''-OH

in welcher GPIR-la'' wie oben definiert ist und die Hydroxylgruppe in den Tyrosinen des Restes GPIR-la'' fehlendes phenolisches Hydroxyl ist, mit einer Verbindung der Formel

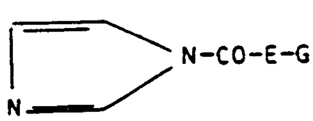

VI

worin E und G wie oben definiert sind, bei einer Temperatur von 10 bis 40 °C in einem wässerigen Lösungsmittel, das gegebenenfalls ein mit Wasser mischbares organisches Lösungsmittel enthält, reagieren gelassen wird.

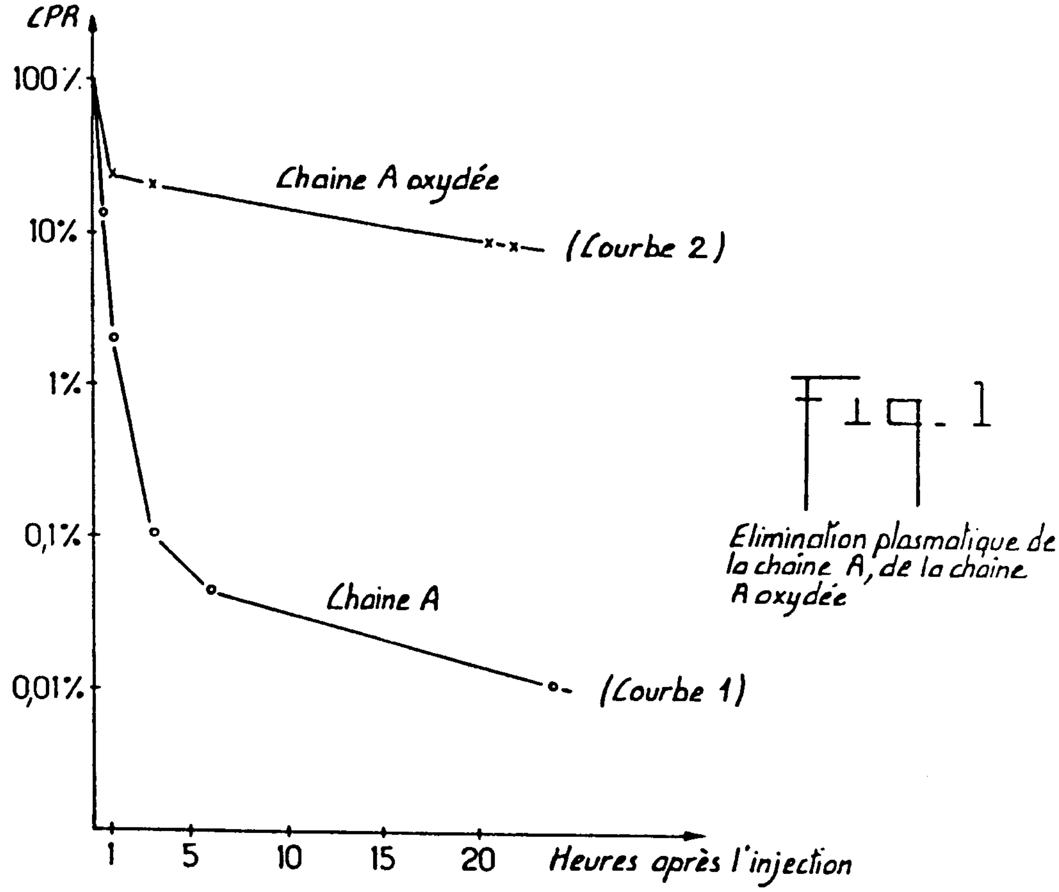


Fig. 1

Elimination plasmatique de la chaine A, de la chaine A oxydée

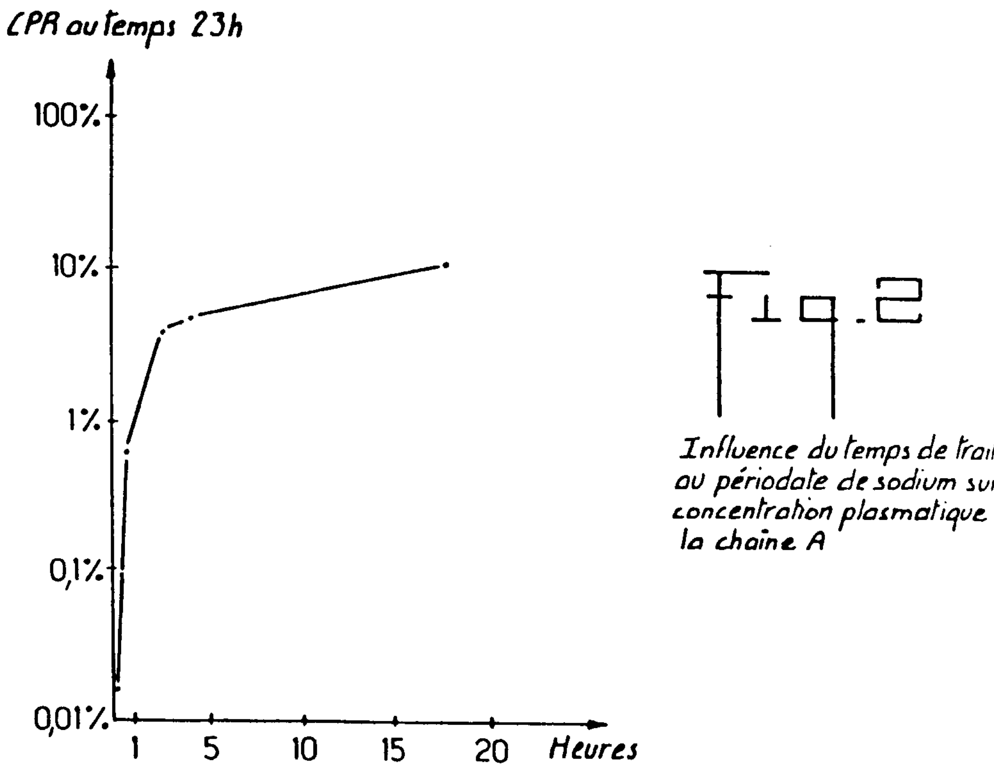


Fig. 2

Influence du temps de traitement au périodate de sodium sur la concentration plasmatique de la chaine A

0192002

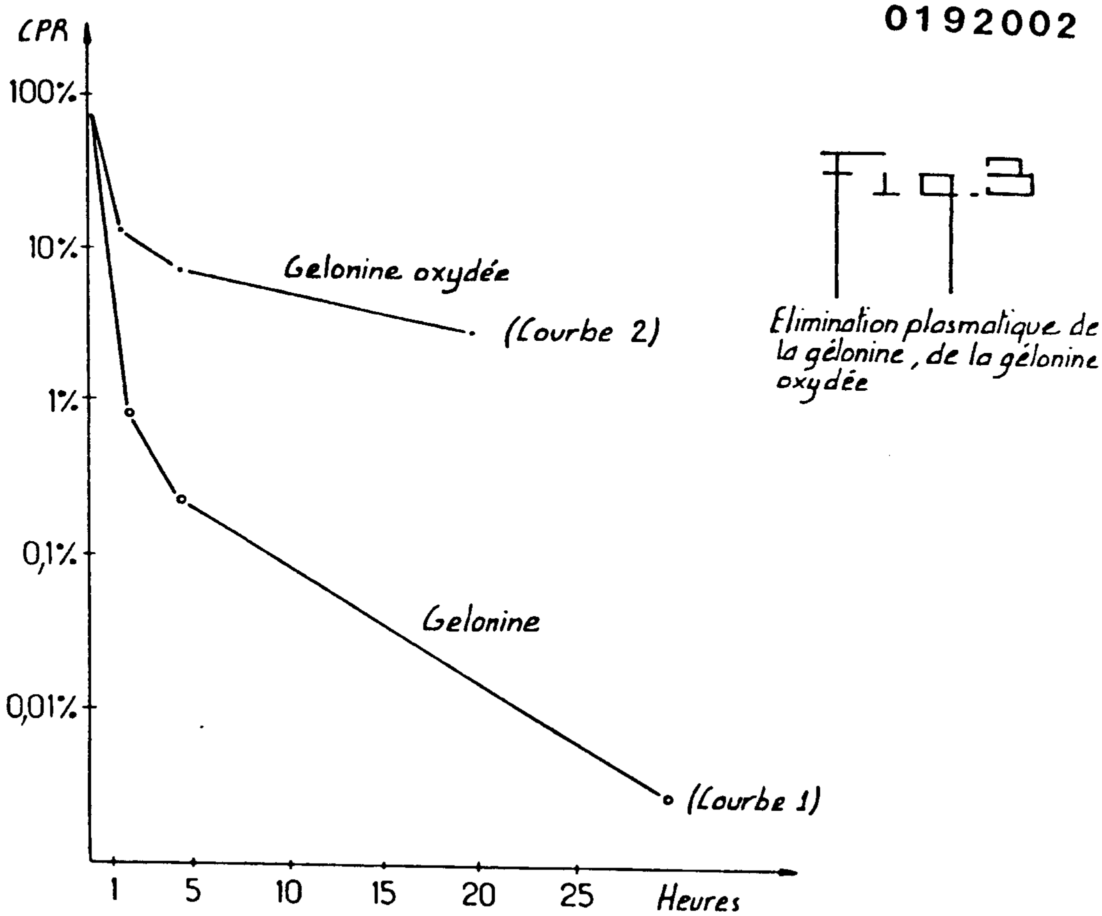


Fig.3

Elimination plasmatique de la gélonine, de la gélonine oxydée

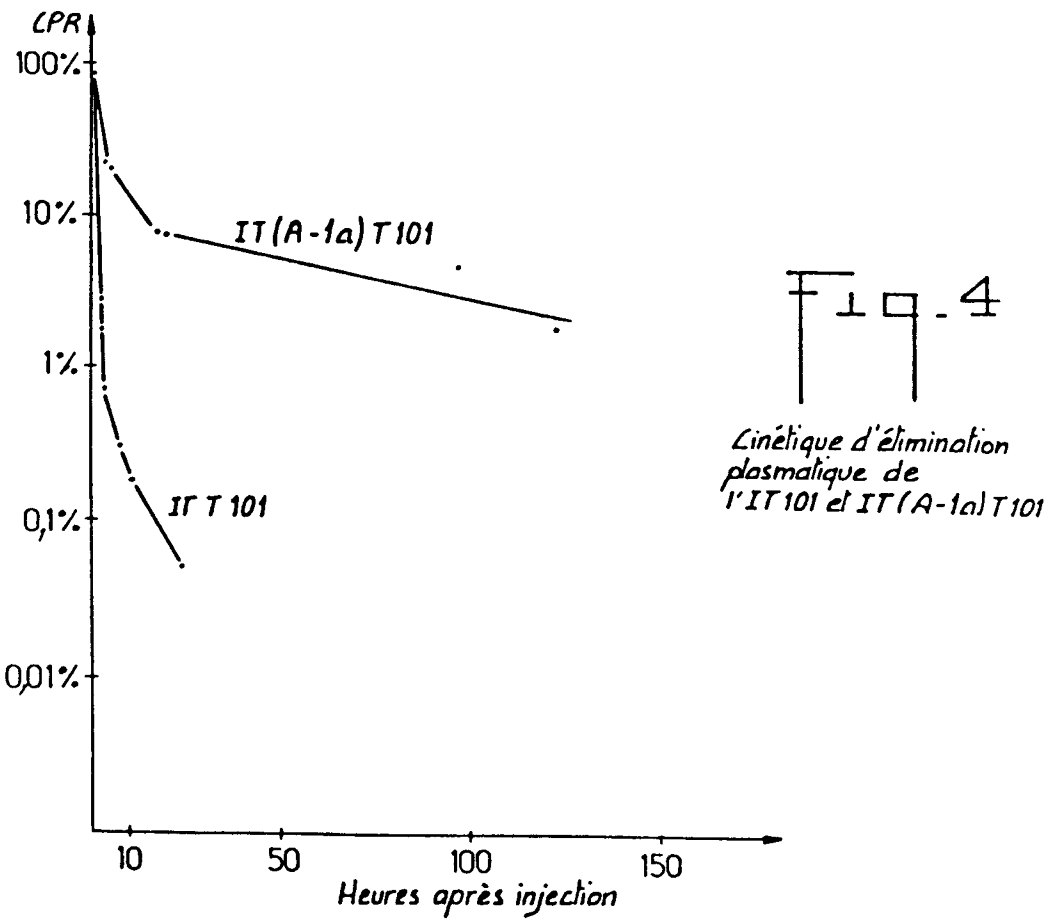


Fig.4

Cinétique d'élimination plasmatique de l'IT 101 et IT (A-1a) T 101

Fig.5

Cytotoxicité de l'IT (A-1a) T101 et de (A-1a) sur les cellules CEM mesurée par l'inhibition de la synthèse proteique

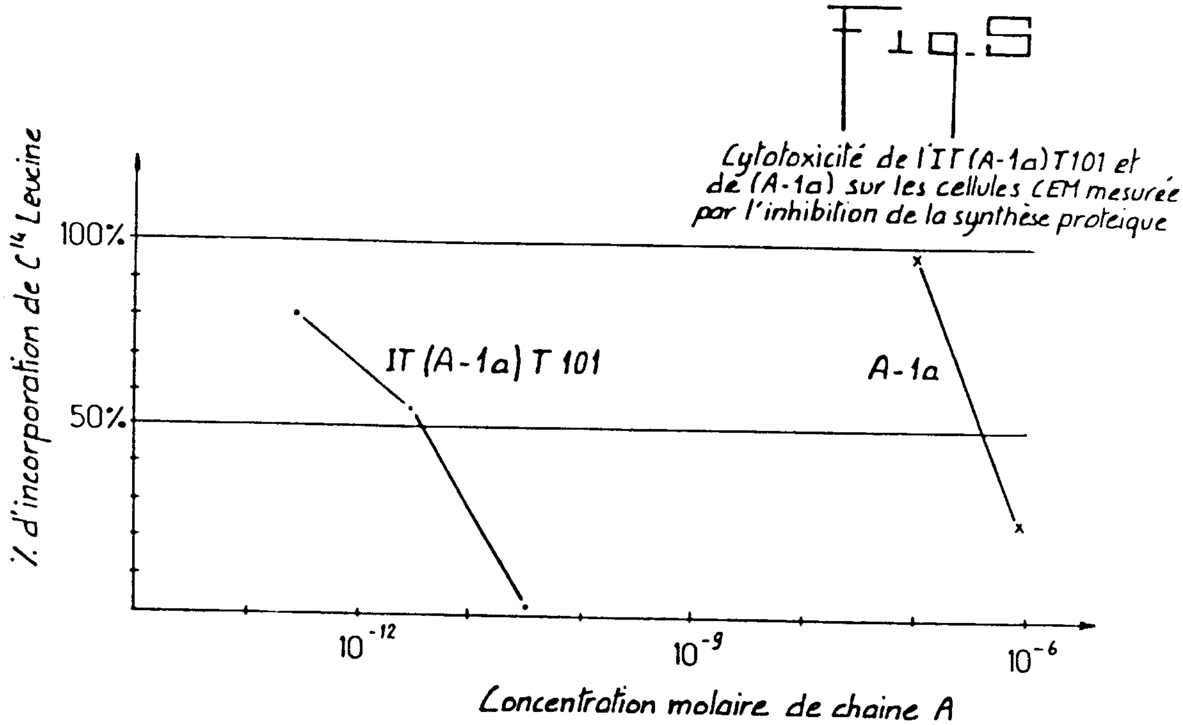


Fig.6

Cytotoxicité de l'IT (A-1a) T101 et de l'IT T101 sur les cellules CEM mesurée en test clonogénique